# EUROPEAN PATENT APPLICATION

(11) **EP 3 546 948 A1**
(43) Date of publication of application: **02.10.2019**
(21) Application number: 19164567.0
(22) Date of filing: 22.03.2019
(51) Int. Cl.: G01N 33/68

(54) **METHOD FOR IDENTIFYING AND ANALYSING MICROVESICLES IN A BIOLOGICAL FLUID SAMPLE**

(30) Priority: 26.03.2018 IT 201800003981
(71) Applicant: Marchisio, Marco, 65019 Pianella (PE) (IT); Lanuti, Paola, 66010 Roccamontepiano (CH) (IT); Miscia, Sebastiano, 66023 Francavilla al Mare (CH) (IT); Simeone, Pasquale, 72019 San Vito del Normanni (BR) (IT); Pierdomenico, Laura, 65019 Pianella (PE) (IT); Bologna, Giuseppina, 66023 Francavilla al Mare (CH) (IT); Ercolino, Eva, 65128 Pescara (PE) (IT)
(72) Inventor: Marchisio, Marco, 65019 Pianella (PE) (IT); Lanuti, Paola, 66010 Roccamontepiano (CH) (IT); Miscia, Sebastiano, 66023 Francavilla al Mare (CH) (IT); Simeone, Pasquale, 72019 San Vito del Normanni (BR) (IT); Pierdomenico, Laura, 65019 Pianella (PE) (IT); Bologna, Giuseppina, 66023 Francavilla al Mare (CH) (IT); Ercolino, Eva, 65128 Pescara (PE) (IT)
(74) Representative: Fraire, Cristina

(57) **Abstract**

The present invention relates on an *ex vivo* method for the analysis, enumeration and/or classification and/or isolation of microvesicles present in whole biological fluid samples, in particular by flow cytometry.

## Description

### FIELD OF THE INVENTION

The object of the present invention is an *ex vivo* method for the identification, enumeration, classification and/or isolation by means of instrumental cell sorting of the circulating microvesicles present in a biological fluid sample, for example blood, plasma, tears, saliva, cerebrospinal fluid, synovial fluid, ascitic tumour or urine, in particular by flow cytometry techniques.

### TECHNICAL BACKGROUND

Circulating microvesicles, also called circulating microparticles, are small non-nucleated particles with sizes ranging from 0.1 to 1 µm, released in biological fluids potentially from all cell types, for example from hematopoietic cells, endothelial cells, epithelial cells, neural cells, glial cells, tumour cells, upon physiological or pathological stimuli.

Microvesicles are characterized by an intact plasma membrane and by a biological activity. It has also been observed that they carry intracellular organelles, such as mitochondria (Tramontano et al., Mediators of Inflammation, 2010 (2010: 250476); Boudreau LH et al., Blood, 2014; 124 (14): 2173 -83).

Microvesicles express surface antigens typical of the cells from which they originate, therefore it is possible, by their antigen identification, to classify them as sub-populations according to their origin. For example, microvesicles deriving from erythrocytes express Glycophorin A (CD235a) (Azza AG Tantaway et al., European Journal of Haematology, 90 (508-518), 2013)), platelet-derived microvesicles express CD41a and P-selectin (CD62P) (Fariborz Mobarrez et al., Thrombosis Research, 2010; 125: 110-116), microvesicles deriving from leukocytes express CD45 (Rosa Suades et al., Thrombosis and Haemostasis 2014; 111: 111-121), microvesicles arising from endothelial cells do not express CD45 and express CD31, CD34 and CD146 (M.Diamant et al., European Journal of Clinical Investigation, 2004; 34: 392-401; Raskit Lachmann et al., Cytometry part A, 2012; 81a: 549- 551).

Recent studies have shown that microvesicles contribute to the functional regulation of different organs and tissues (Ritu Jaiswall et al, PLoS One, 2013; 8 (4): e61515) and represent the activation and/or tissue degeneration index linked to pathophysiological events, as, for example, cardiovascular diseases, diabetes, atherosclerosis, tumours, autoimmune and inflammatory disorders (Dilyana Todorova et al, Circ Res., 2017; 120:1658-1673).

Therefore, methods for their identification, quantification and classification in biological samples from a subject are of considerable interest as potential diagnostic and prognostic tools and for the evaluation of the response to treatment in many pathologies (Bence Györy et al, Cellular and Molecular Life Sciences, 2011; 68: 2667-2688 and Kenneth W. Witwer et al., Journal of Extracellular Vesicles, 2013; 2: 20360 -20385).

Methods for the identification of microvesicles in peripheral blood samples through the use of flow cytometry techniques based on optical refractive parameters are described in the literature.

All these methods require that blood samples that have to be analysed undergo a pre-analytical treatment step, which involves centrifugation and/or ultracentrifugation and/or freezing or lysing steps, in order to enrich the microvesicle component and/or eliminate cellular elements that could interfere with the techniques used for the identification. However, it has been observed that sample manipulation in the pre-analytical phase leads to artefact production and poor reproducibility.

Furthermore, it has been demonstrated that flow cytometry methods for the identification of microvesicles based only on the measurement for each single particle of forward scattering (FSC) and/or side scattering (SSC) parameters, lead to an underestimation of the number of circulating microvesicles (W.L. Chandler et al, J Thromb Haemost, 2011; 9: 1216-24).

These limitations have therefore hindered the development of those methods to the clinical practice.

There is therefore the need of developing new methods that allow the analysis of microvesicle component directly on biological samples, for example whole blood, avoiding any pre-analytical manipulation procedure and leading to reproducible and precise results.

### SUMMARY OF THE INVENTION

The present inventors have found a combination of fluorescent markers that together with morphological parameters allow to quantitatively and specifically identify the microvesicle compartment directly on whole biological fluid samples, avoiding any manipulation procedure before the analysis.

In particular, the present inventors have surprisingly found that a series of fluorescent reagents commonly used as mitochondria markers in living cells are able to accumulate into microvesicles present in biological samples independently from the presence of mitochondria therein. Moreover, among the reagents commonly used to stain mitochondria, only cationic cyanins and cationic carbocyanines with an asymmetric structure, having the below described general formula, are able to stain microvesicles, allowing to distinguish them from debris and other corpusculated elements present in the blood.

Therefore, a first object of the present invention is a method for the identification of microvesicles, either containing or not mitochondria, present in a biological fluid sample, preferably whole, isolated from a subject, comprising the following steps:
a) contacting said sample with at least the following fluorescent reagents:
   - a first fluorescent reagent, consisting of a mitochondrial fluorescent reagent, preferably potentiometric, having the formula (I): 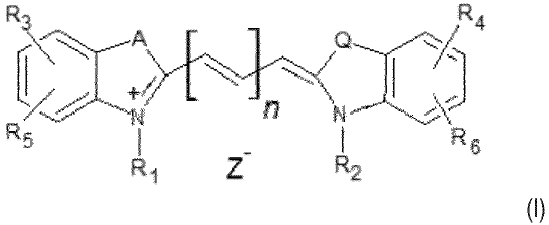
      wherein n is 0 or an integer between 1 and 3;
      R₁ and R₂ are different from each other and selected from among H and organic substituents with a molecular weight lower than 300 g/mol;
      A and Q are independently selected from among O, S, Se, C (R₇R₈), CH = CH, NR₉, wherein R₇, R₈ and R₉ are independently selected from among H and organic substituents with a molecular weight lower than 300 g/mol; R₃, R₄, R₅ and R₆ are independently selected from among H, organic substituents of molecular weight lower than 300 g/mol or R₃ and R₄ and/or R₅ and R₆ are combined to form an additional aromatic or heteroaromatic ring; preferably a benzene ring.
      Z- is selected from among halides (Cl⁻, Br⁻, I⁻) and ClO₄⁻; and
   - a second fluorescent reagent selected from among phalloidin and an antibody directed against CD235a conjugated to a fluorochrome group;
b) analysing the treated sample obtained in step a) and identifying the particles contained in the sample which have dimension smaller than 1 µm, preferably between 0.1 to 1 µm, positive to the detection of a fluorescence signal emitted by the aforementioned first fluorescent reagent, and negative for the fluorescence signal emitted by the aforementioned second fluorescent reagent, thereby identifying microvesicles, either containing or not mitochondria, present in the sample..

A second object of the present invention is a method for the prognosis, diagnosis and/or evaluation of the stage of a pathology in a subject comprising the identification, enumeration and/or classification of microvesicles in a biological fluid sample, preferably whole, obtained from a subject through the method according to the first object of the invention and the subsquent correlation of the number of microvesicles present into the sample and/or the presence and or the quantity of one or more microvesicle subpopulations into the sample with a diagnosis, prognosis and/or a specific pathology stage.

The aforementioned method according to the second object of the present invention can also be used to evaluate a patient's response to a pharmacological treatment, through the identification, enumeration and/or classification of the microvesicles in a biological fluid, preferably whole, isolated from the subject before and after the treatment and by evaluating whether the variation of the number of microvesicles from a sample and/or of the presence and/or the quantity of one or more microvesicle subpopulations is related to a specific diagnosis or a disease stage.

A third object of the present invention is a kit for the identification of microvesicles, containing or not containing mitochondria, present in a sample of a biological fluid, preferably whole, isolated from a subject, comprising at least:
- a first fluorescent reagent, consisting of a mitochondrial fluorescent reagent, preferably potentiometric, having the formula (I): 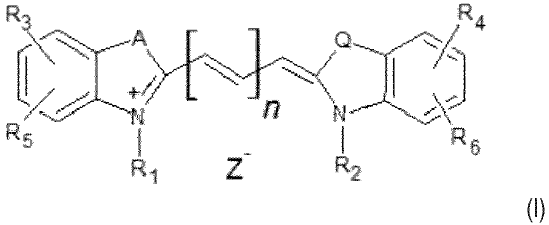
   wherein n is 0, or an integer between 1 and 3;
   R₁ and R₂ are different from each other and selected from among H and organic substituents with a molecular weight lower than 300 g/mol;
   A and Q are independently selected from among O, S, Se, C(R₇R₈), CH = CH, NR₉, wherein R₇, R₈ and R₉ are independently selected from among H and organic substituents with a molecular weight lower than 300 g/mol. R₃, R₄, R₅ and R₆ are independently selected from among H, organic substituents of molecular weight lower than 300 g/mol or R₃ with R₄ and/or R₅ with R₆ are combined to form an additional aromatic or heteroaromatic ring, preferably a benzene ring;
   Z- is an ion selected from among Cl⁻, Br⁻, I⁻, ClO₄⁻; and
- a second fluorescent reagent, selected from among a phalloidin and an antibody directed against CD235a conjugated to a fluorochrome group.

### FIGURE DESCRIPTION

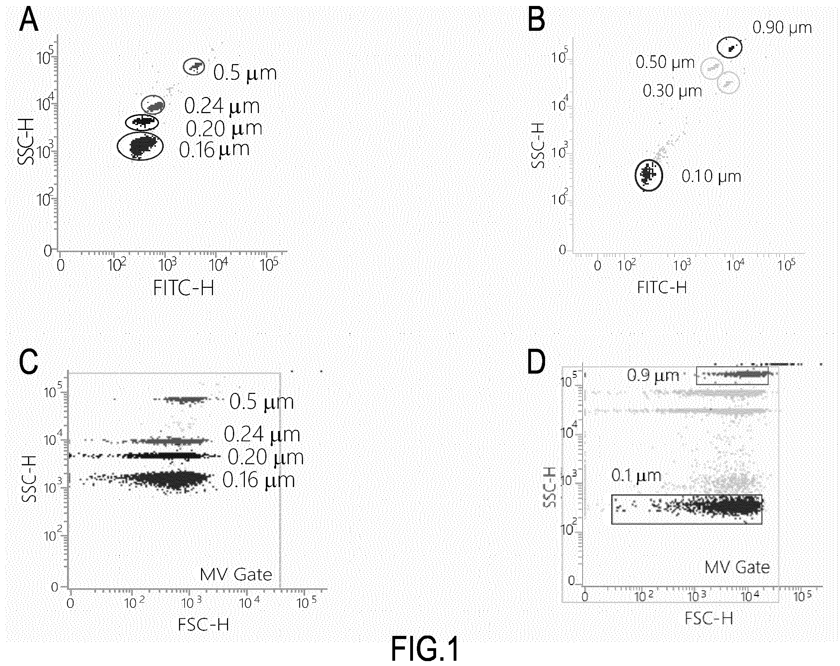

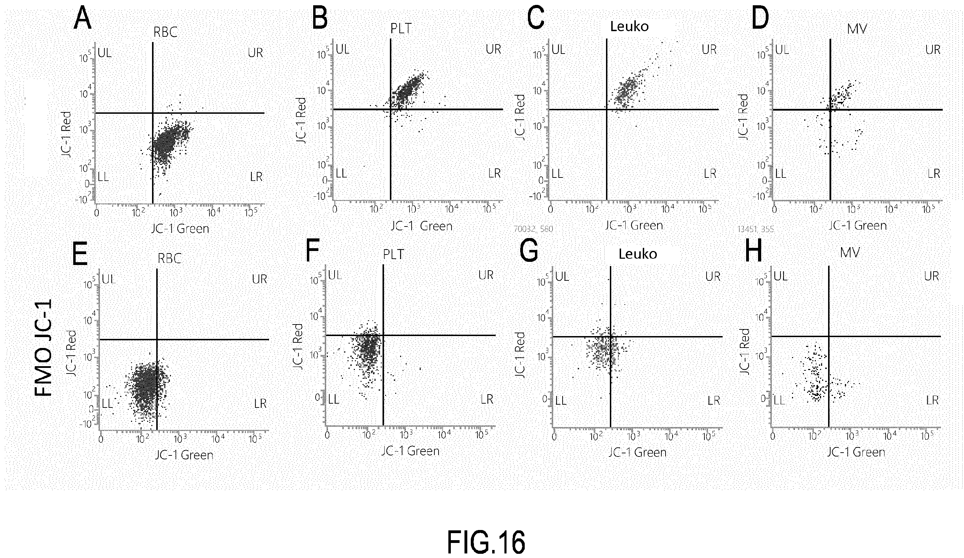

Figure 1 shows the dot plots obtained from the flow cytometry acquisition of the Megamix Plus SSC and FSC beads, as described in Example 1. In detail, Figure 1A shows, on a SSC-H/FITC-H dot-plot the Megamix-Plus SSC beads in which each population of beads, of 0.16, 0.20, 0.24 and 0.50 µm size, has been gated separately;
Figure 1B shows a SSC-H/FITC-H dot-plot in which each population of Megamix Plus FSC beads, of 0.10, 0.30, 0.50 and 0.90 µm size, has been gated separately; Figure 1C shows, Megamix Plus SSC beads of 0.16, 0.20, 0.24 and 0.50 µm size on a SSC-H/FSC-H dot-plot; Figure 1D shows Megamix Plus FSC beads on a SSC-H/FSC-H dot-plot, in particular 0.10 and 0.90 µm populations of beads have been highlighted (those are the beads whose dimensions allow to identify microvesicles). It has been defined the region identified as "MV gate" (Figure 1C-D), comprising all the Megamix Plus SSC and all the Megamix Plus FSC beads.

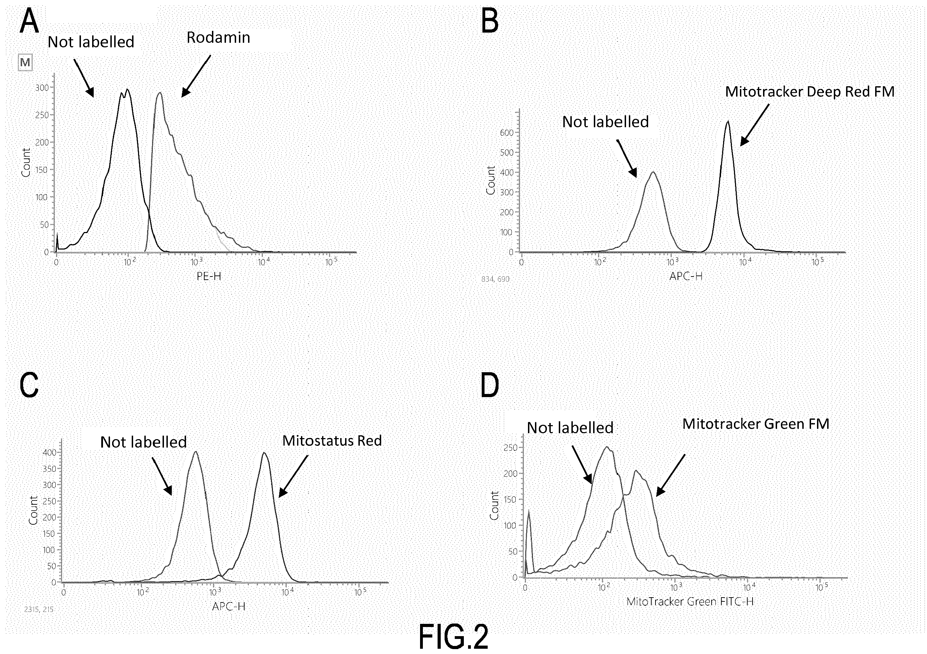

Figure 2 shows the fluorescence profile of liposomes labelled with Rhodamine (Figure 2A), Mitotracker Deep Red FM (Figure 2B), Mitostatus Red (Figure 2C) and Mitotracker Green FM (Figure 2D), compared to the same unstained liposomes (unstained curves), as described in Example 2.

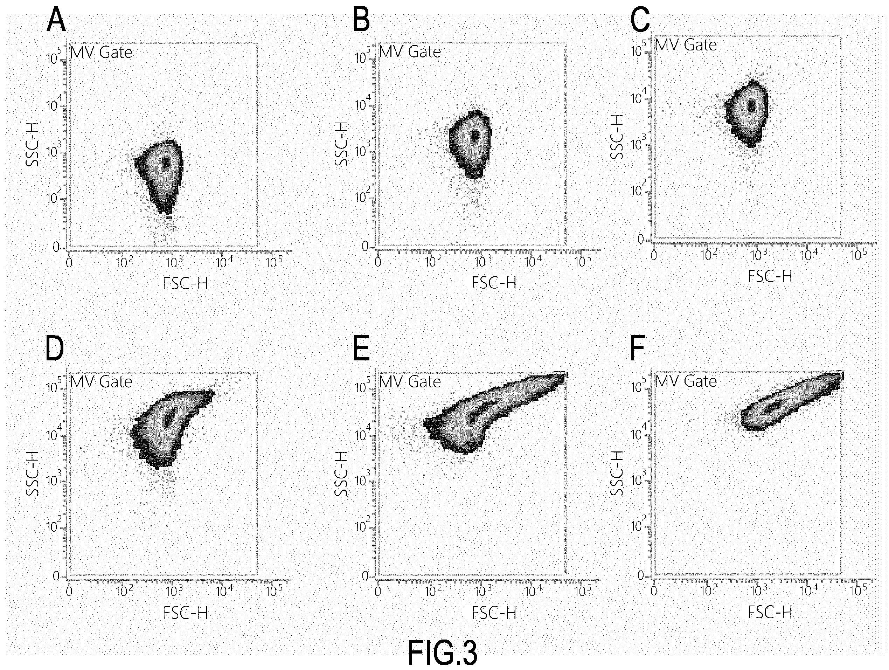

Figure 3 shows, on an SSC-H/FSC-H dot-plot, liposomes of different sizes: 50 nm (Figure 3A), 100 nm (Figure 3B), 200 nm (Figure 3C), 400 nm (Figure 3D), 600 nm (Figure 3E), 1 µm (Figure 3F), acquired with the same instrumental settings used to record Megamix Plus beads, as described in Example 2.

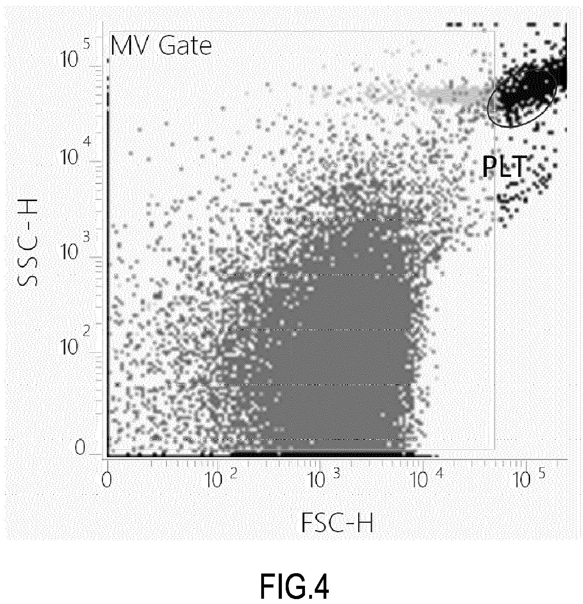

Figure 4 shows, on a SSC-H/FSC-H dot-plot, the population of events characterized by scatter parameters in the "MV gate" region, obtained from the whole peripheral blood sample from a healthy subject, as described in Example 3.

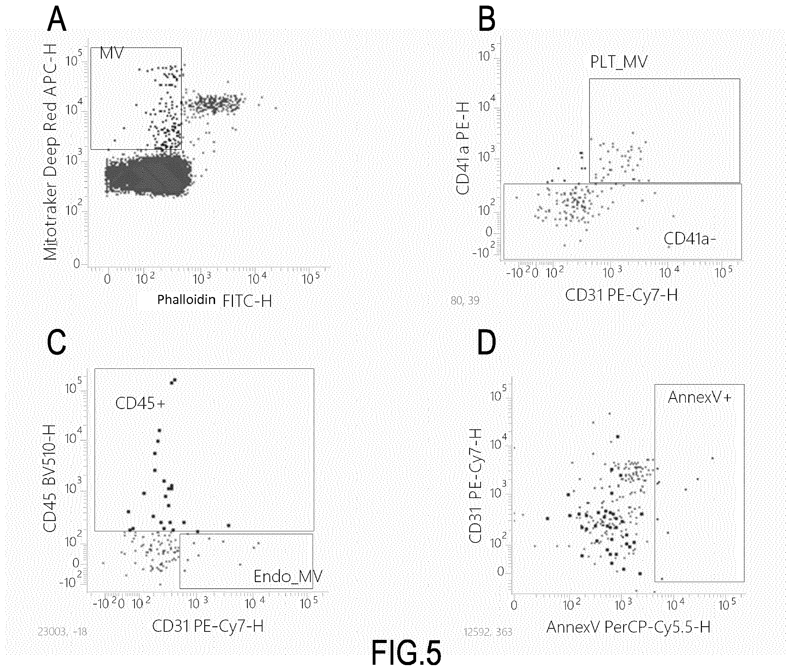

Figure 5 shows the gating strategy used to identify the different microvesicle compartments present in a peripheral blood sample stained by the panel reported in Table 1, as described in Example 4. In particular, Figure 5A shows the events obtained from the analysis of a peripheral blood sample, analysed on a Phalloidin FITC-H/MitoTracker Deep Red FM-APC-H dot-plot, on which the events previously identified in the "MV gate" region are shown, and total microvesicles (events positive to the MitoTracker Deep Red FM and negative to the phalloidin) have been then identified. Figure 5B shows total microvesicles on a CD31 PE-Cy7-H/CD41a PE-H dot-plot, on which platelet-derived microvesicles (PLT_MV, CD41a+/CD31+) and CD41a- microvesicles have been identified; Figure 5C shows CD41a- microvesicles on a CD31 PE-Cy7-H/CD45 BV510-H dot-plot, on which microvesicles from endothelial origin (Endo_MV, CD45-/CD31+) and microvesicles from leukocyte origin (CD45+) have been identified; in Figure 5D total microvesicles (MV) have been shown on an Annexin V PerCP-Cy5.5-H/CD31 PE-Cy7-H dot-plot.

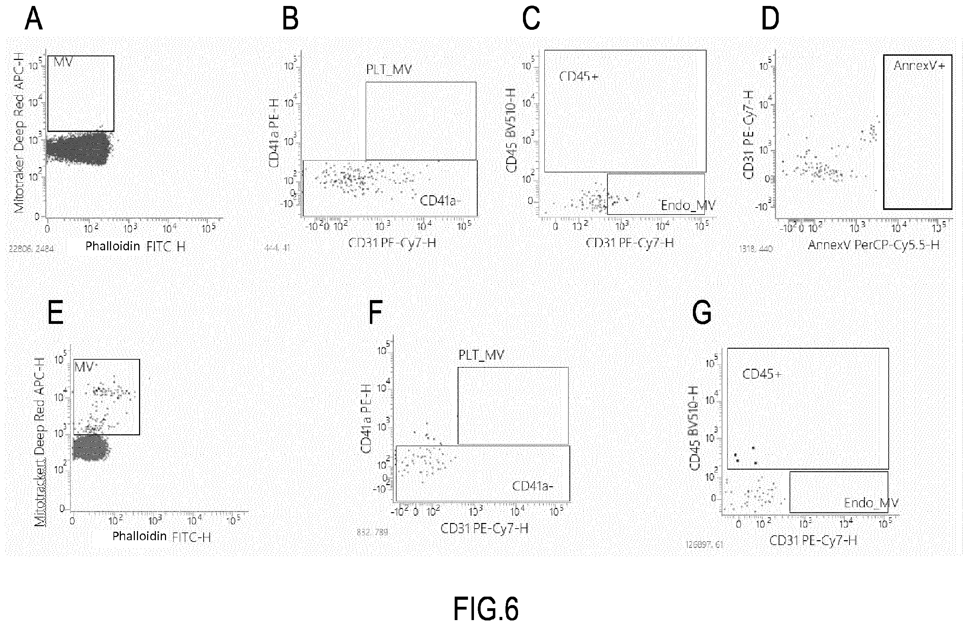

In Figure 6 are represented control samples for the different reagents/antibodies fluorescence of the Table 1 panel, as described in Example 4. Figure 6A represents the fluorescence control for MitoTracker Deep Red FM, obtained by representing on a Phalloidin FITC-H/MitoTracker Deep Red FM-APC-H dot-plot, the events previously identified in the respective "MV gate" from a peripheral blood sample stained by the panel described in Table 1 and then treated by 1% Triton X-100 detergent for one hour. Figure 6B represents a CD31 PE-Cy7-H/CD41a PE-H dot-plot on which total microvesicles from the FMO sample (referred to the panel described in Table 1) for the CD41a PE are shown. Figure 6C represents a CD31 PE-Cy7-H/CD45 BV510-H dot-plot on which CD41a- microvesicles from the FMO sample for CD45 BV510 are shown. Figure 6D represents on an Annexin V PerCP-Cy5.5-H/CD31 PE-Cy7-H dot-plot on which total microvesicles from the FMO sample (referred to the panel reported in Table 1) for Annexin V PerCP-Cy5.5 are shown. Figure 6E represents a Phalloidin FITC-H/MitoTracker Deep Red FM-APC-H dot-plot on which, in the MV gate, total microvesicles from the FMO sample (referred to the panel reported in Table 1) for Phalloidin-FITC are shown. Figure 6F represents a CD31 PE-Cy7-H/CD41a PE-H dot-plot on which CD41a- microvesicles from the FMO sample (referred to the panel described in Table 1) for CD31 PE-Cy7 are shown. Figure 6G represents a CD31 PE-Cy7-H/CD45 BV510-H dot-plot on which CD41a- microvesicles from the FMO sample (referred to the panel described in Table 1) for CD31 PE-Cy7 are shown.

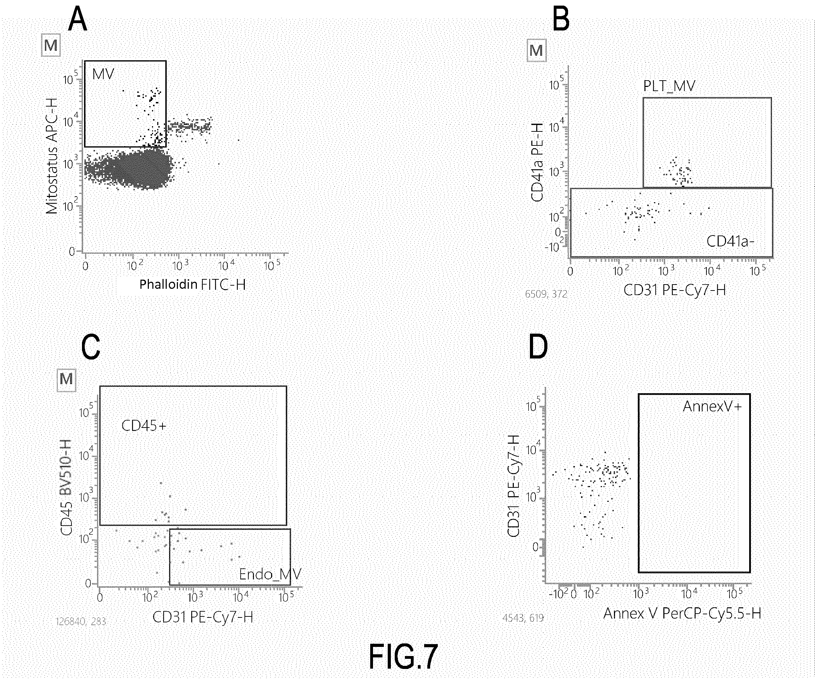

Figure 7 shows the gating strategy used to identify the different microvesicle compartments present in a peripheral blood sample stained by the panel reported in Table 2, as described in Example 5. Figure 7A shows the events obtained from the analysis of a peripheral blood sample, analysed on a Phalloidin FITC-H/MitoStatus-APC-H dot-plot, on which the events previously identified in the "MV gate" region are shown, and total microvesicles (events positive to the MitoStatus and negative to the phalloidin) have been then identified. Figure 7B shows total microvesicles on a CD31 PE-Cy7-H/CD41a PE-H dot-plot, on which platelet-derived microvesicles (PLT_MV, CD41a+/CD31+) and CD41a- microvesicles have been identified; Figure 7C shows CD41a- microvesicles on a CD31 PE-Cy7-H/CD45 BV510-H dot-plot, on which microvesicles from endothelial origin (Endo_MV, CD45-/CD31+) and microvesicles from leukocyte origin (CD45+) have been identified; in Figure 7D total microvesicles (MV) have been shown on an Annexin V PerCP-Cy5.5-H/CD31 PE-Cy7-H dot-plot.

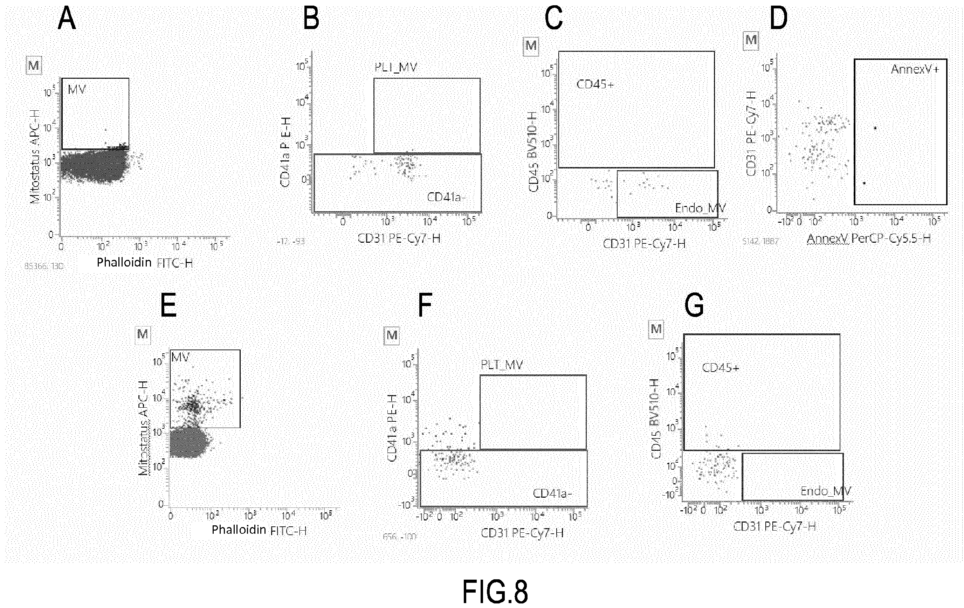

In Figure 8 are represented control samples for the fluorescence of the different reagents/antibodies of the Table 2 panel, as described in Example 5. Figure 8A represents the fluorescence control for MitoStatus, obtained by representing on a Phalloidin FITC-H/MitoStatus-APC-H dot-plot, the events previously identified in the respective "MV gate" of a peripheral blood sample stained by the panel described in Table 2 and then treated by 1% Triton X-100 detergent for one hour. Figure 8B represents a CD31 PE-Cy7-H/CD41a PE-H dot-plot on which total microvesicles from the FMO sample (referred to the panel described in Table 2) for CD41a PE are shown. Figure 8C represents a CD31 PE-Cy7-H/CD45 BV510-H dot-plot on which CD41a- microvesicles from the FMO sample (referred to the panel described in Table 2) for CD45 BV510 are shown. Figure 8D represents on an Annexin V PerCP-Cy5.5-H/CD31 PE-Cy7-H dot-plot on which total microvesicles from the FMO sample (referred to the panel reported in Table 2) for Annexin V PerCP-Cy5.5 are shown. Figure 8E represents a Phalloidin FITC-H/MitoStatus-APC-H dot-plot on which total microvesicles from the FMO sample (referred to the panel reported in Table 2) for Phalloidin-FITC are shown. Figure 8F represents a CD31 PE-Cy7-H/CD41a PE-H dot-plot on which CD41a- microvesicles from the FMO sample (referred to the panel described in Table 2) for CD31 PE-Cy7 are shown. Figure 8G represents a CD31 PE-Cy7-H/CD45 BV510-H dot-plot on which CD41a-microvesicles from the FMO sample (referred to the panel described in Table 2) for CD31 PE-Cy7 are shown.

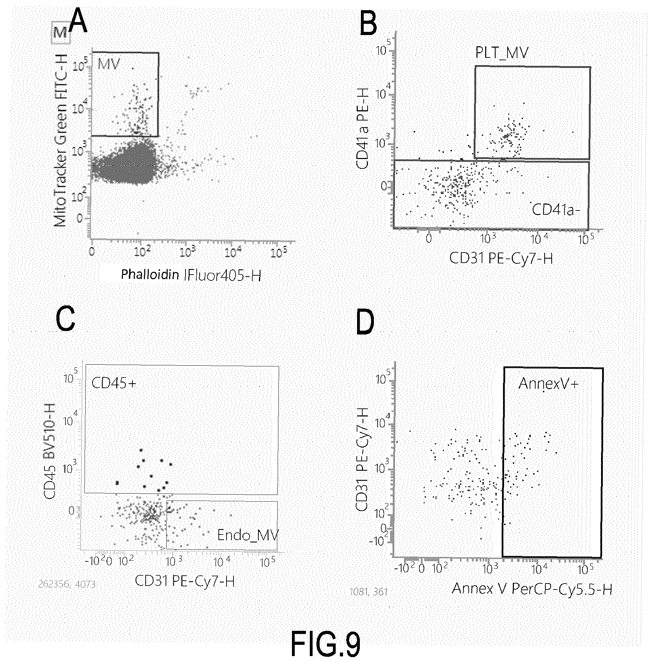

Figure 9 shows the gating strategy used to identify the different microvesicle compartments present in a peripheral blood sample stained by the reagent/antibody panel reported in Table 3, as described in Example 6. Figure 9A shows the events obtained from the analysis of a peripheral blood sample, analysed on an iFluor405-H/MitoTracker Green FM FITC-H dot-plot, on which the events previously identified in the "MV gate" region are shown, and total microvesicles (events positive to the MitoTracker Green FM and negative to the phalloidin) have been then identified. Figure 9B shows total microvesicles on a CD31 PE-Cy7-H/CD41a PE-H dot-plot, on which platelet-derived microvesicles (PLT_MV, CD41a+/CD31+) and CD41a- microvesicles have been identified; Figure 9C shows CD41a- microvesicles on a CD31 PE-Cy7-H/CD45 BV510-H dot-plot, on which microvesicles from endothelial origin (Endo_MV, CD45-/CD31+) and microvesicles from leukocyte origin (CD45+) have been identified; in Figure 9D total microvesicles (MV) have been shown on an Annexin V PerCP-Cy5.5-H/CD31 PE-Cy7-H dot-plot.

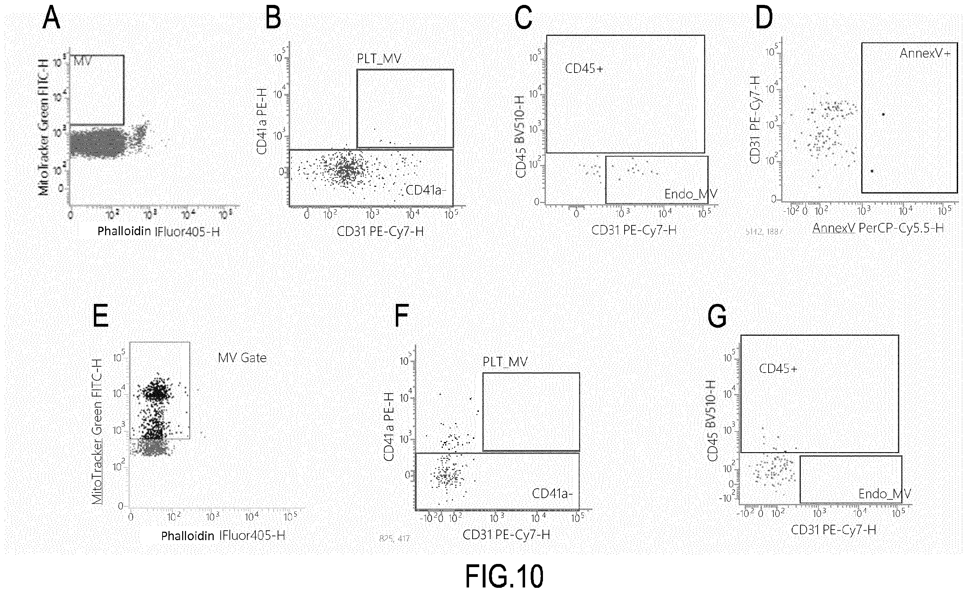

In Figure 10 are represented the control samples for the different reagent/antibody fluorescence of the panel described in Table 3, as described in Example 6. Figure 10A represents the fluorescence control for MitoTracker Green FM, obtained by representing on a Phalloidin iFluor405-H/MitoTracker Green-FITC-H dot-plot, the events previously identified in the respective "MV gate" from a peripheral blood sample stained by the panel described in Table 3 and then treated with 1% Triton X-100 detergent for one hour. Figure 10B represents a CD31 PE-Cy7-H/CD41a PE-H dot-plot on which total microvesicles from the FMO sample (referred to the panel described in Table 3) for CD41a PE are shown. Figure 10C represents a CD31 PE-Cy7-H/CD45 BV510-H dot-plot on which CD41a- microvesicles from the FMO sample (relative to the panel described in Table 3) for CD45 BV510 are shown. Figure 10D represents an Annexin V PerCP-Cy5.5-H/CD31 PE-Cy7-H dot plot on which total microvesicles from the FMO sample (referred to the panel described in Table 3) for Annexin V PerCP-Cy5.5 are shown. Figure 10E represents a Phalloidin iFluor405/MitoTracker Green FM dot-plot on which total microvesicles from the FMO sample (referred to the panel described in Table 3) for Phalloidin iFluor405-H are shown. Figure 10F represents a CD31 PE-Cy7-H/CD41a PE-H dot-plot on which CD41a- microvesicles from the FMO sample (referred to the panel described in Table 3) for CD31 PE-Cy7 are shown. Figure 10G represents a CD31 PE-Cy7-H/CD45 BV510-H dot-plot on which CD41a- microvesicles from the FMO sample (referred to the panel described in Table 3) for CD31 PE-Cy7 are shown.

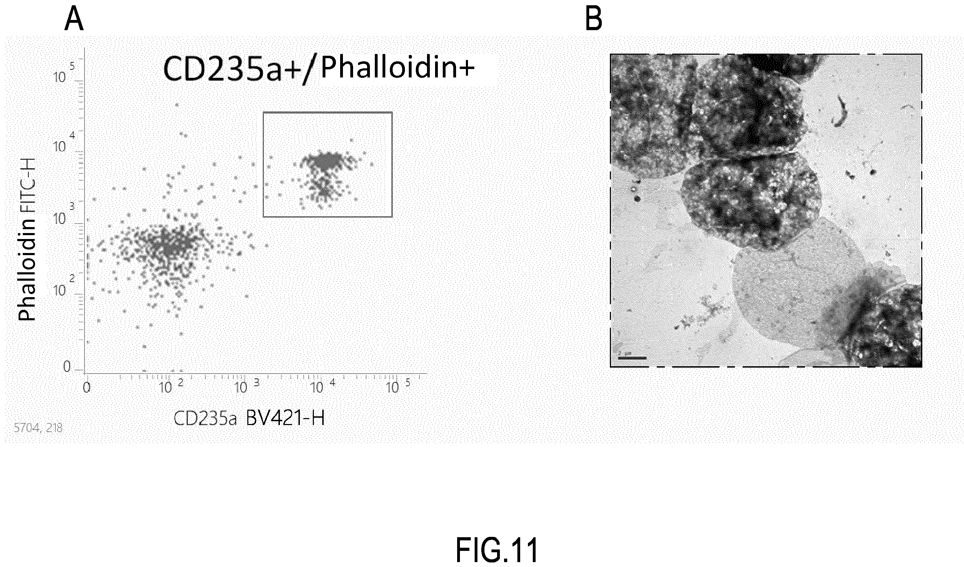

Figure 11 shows, in A, a CD235a BV421-H/Phalloidin FITC-H dot-plot on which the events previously identified in the "MV gate" region from a peripheral blood sample are represented. Events staining simultaneously positive to the CD235a and to the Phalloidin (CD235a+/Phalloidin+) were identified. Figure 11B shows a representative image of pure CD235a+/Phalloidin+ events, obtained by transmission electron microscopy; in Figure 11B is also shown the related scale bar (2 µm). The images are representative of three separate experiments.

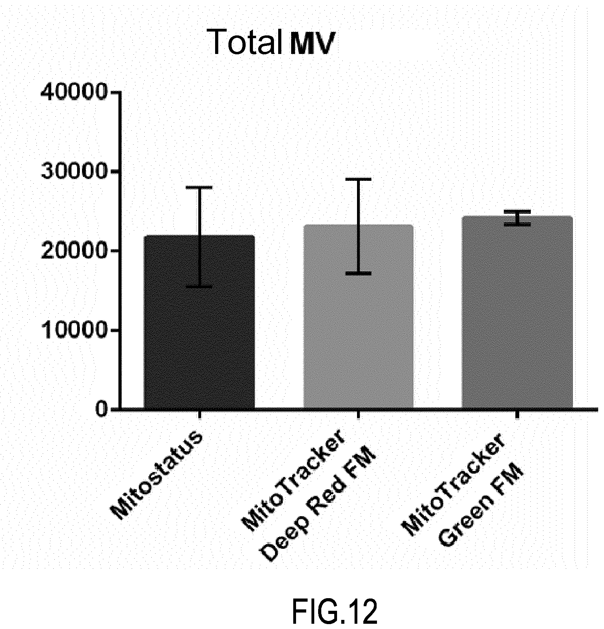

Figure 12 shows the average number of total microvesicles (per µl of whole blood) obtained from 4 different peripheral blood samples stained with the panels described in Tables 2 (MitoStatus), 1 (MitoTracker Deep Red FM) and 3 (MitoTracker Green FM) as described in Examples 5, 4 and 6 respectively. For each bar, the relative standard deviation is shown.

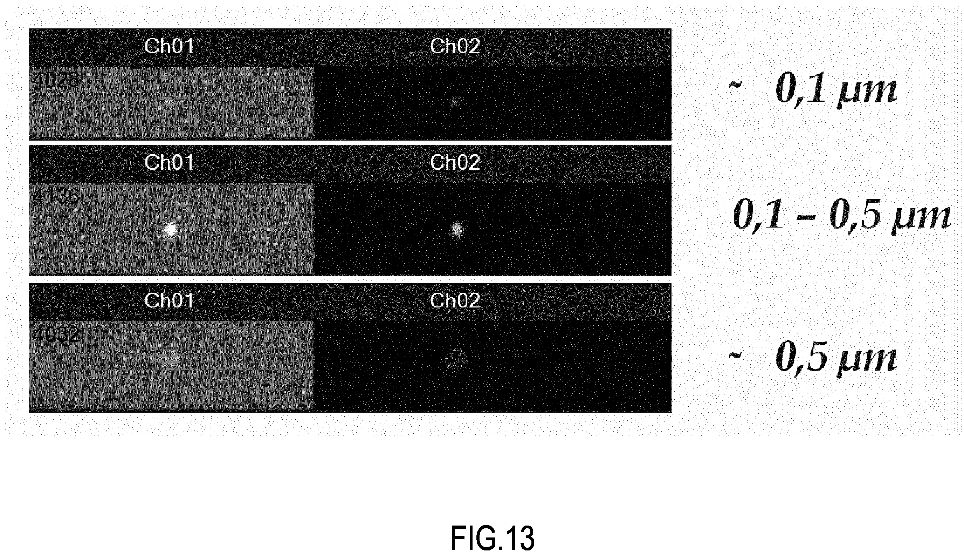

In Figure 13 are shown three representative images of pure microvesicles previously separated by instrumental cell sorting, as described in Example 9, obtained by their ImageStream analysis. Images have been obtained by the 60X magnification. Are shown: on the left a brightfield image, on the right a fluorescence image of the related MitoTracker Green FM staining. The three rows represent 3 different microvesicles, with sizes that progressively increase from the top to the bottom. Images are representative of three separate experiments.

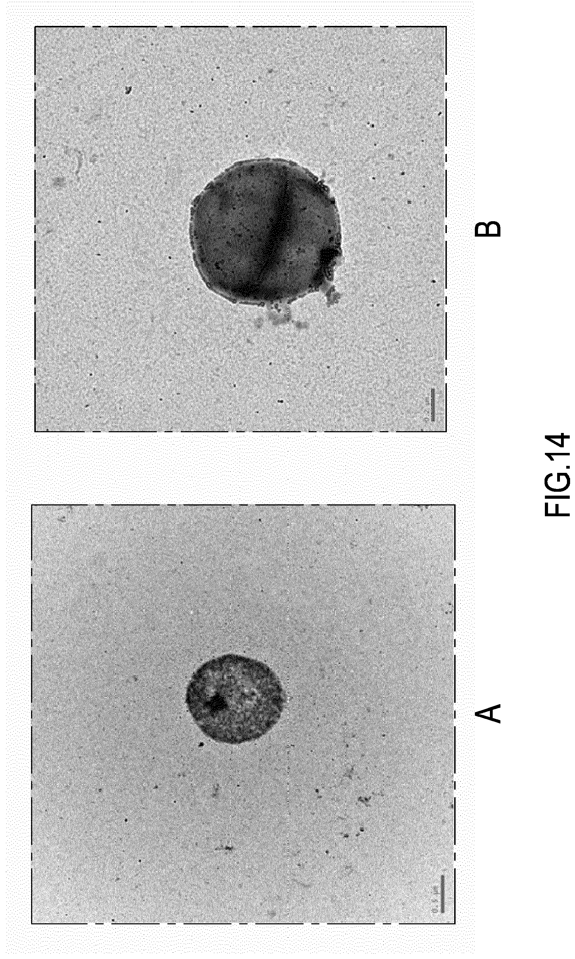

Figure 14 shows two images obtained from the analysis of pure microvesicles (previously separated by instrumental sorting) and analysed by transmission electron microscopy. In Figure 14A the related scale bar (0.5 µm) is shown. In Figure 14B shows the related scale bar (0.2 µm) is shown.

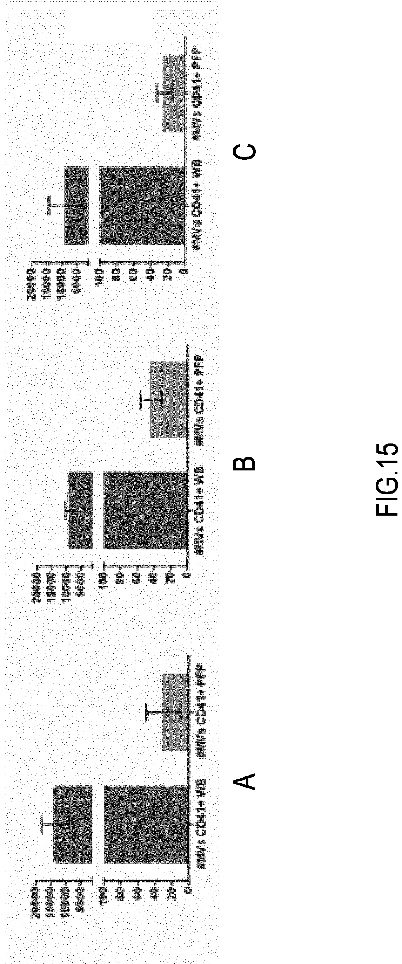

In Figure 15, the bars represent the count of platelet-derived microvesicles (CD41a+) obtained by the three panels indicated in Tables 1-3 (MitoTracker Deep Red FM in A, MitoStatus Red in B, MitoTracker Green FM in C). Each left bar, from each graph, represents the mean number and the standard deviation related to the numbers of PLT_MV obtained from the acquisition of 3 distinct samples of peripheral blood, processed according to Example 4 (Figure 15A), Example 5 (Figure 15B), or Example 6 (Figure 15C). Each right bar from each chart represents the average number and the relative standard deviation of the numbers of PLT_MV obtained from the acquisition of the same 3 distinct samples of peripheral blood above described, processed by the traditional method of separation of the microvesicles (by the preparation of the relative platelet free plasma - PFP samples), as described in the Example 10.
   A-D panels of the Figure 16 show the JC1-Green/JC1-Red dot-plots, representing: red blood cells (RBC, Figure 16A), platelets (PLT, Figure 16B) leukocytes (Leuko, Figure 16C), total microvesicles (MV, Figure 16D). E-H panels of the Figure 16 show the JC1-Green/JC1-Red dot-plots, on which are represented the FMO controls for red blood cells (RBC, Figure 16E), platelets (PLT, Figure 16F), leukocytes (Leuko, Figure 16G), total microvesicles (Figure 16H). The images are representative of three separate experiments.

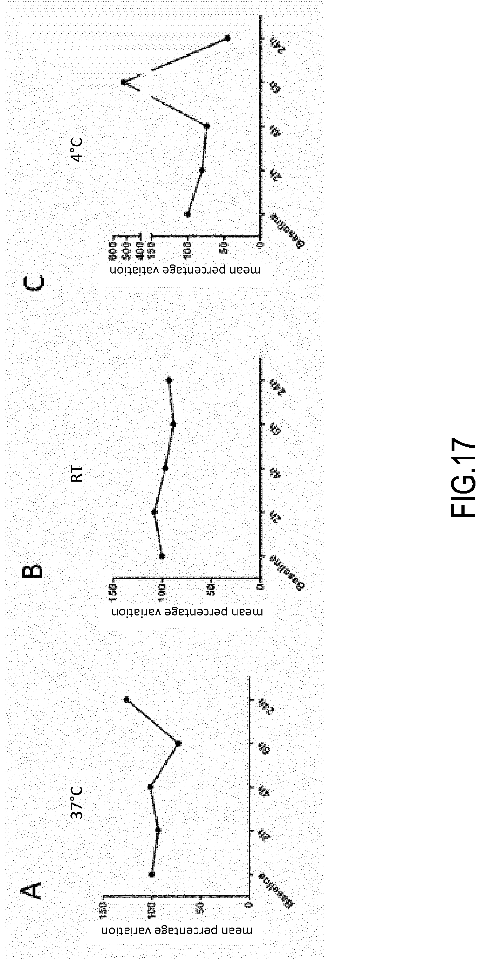

Figure 17 shows the mean percentage deviation (obtained from 3 distinct samples of peripheral blood) of total number of microvesicles at different time points from the collectionl (2h, 4h, 6h, 24h), compared to the microvesicle count at time 0, obtained from samples stored at 37 ° C (Figure 17A), at room temperature (RT, Figure 17B) or at 4 ° C (Figure 17C).

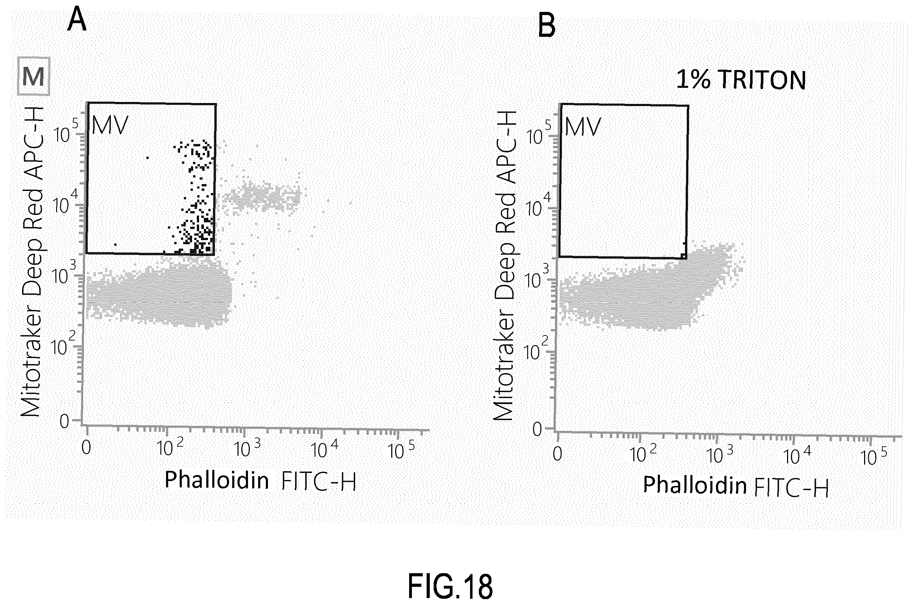

Figure 18 shows a Phalloidin FITC-H/MitoTracker Deep Red FM-APC-H dot-plot in which microvesicles (previously obtained by means of the respective "MV Gate") from a sample stained by the panel described in Table 1 are represented, before (Figure 18A) or after (Figure 18B) the treatment by the Triton X-100 detergent, as described in Example 14. Data are representative of at least 3 separated experiments.

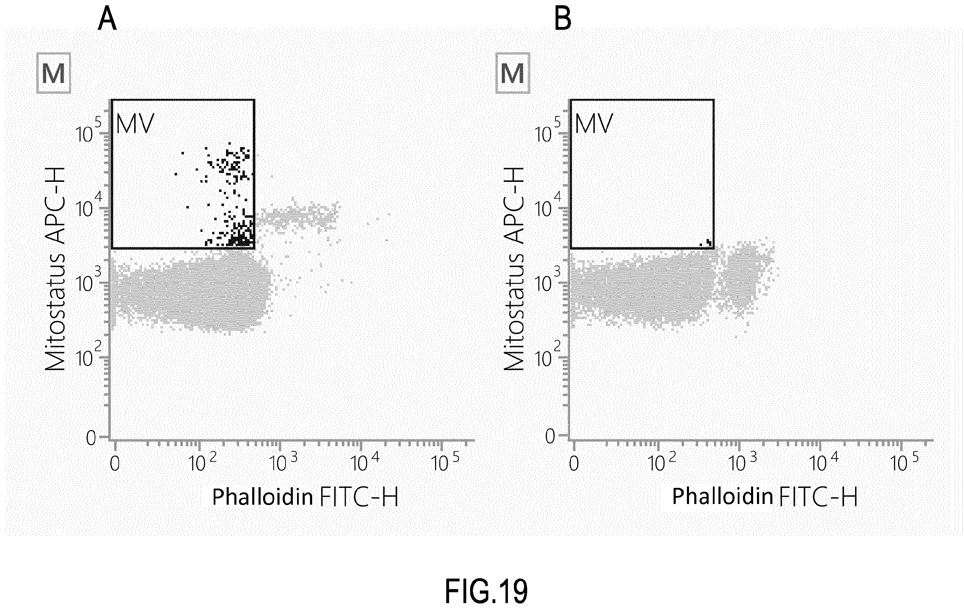

Figure 19 shows a Phalloidin FITC-H/MitoStatus-APC-H dot plot in which microvesicles (previously obtained by means of the respective "MV Gate") from a sample stained by the panel described in Table 2 are represented, before (Figure 19A) or after (Figure 19B) the treatment with the Triton X-100 detergent, as described in Example 14. Data are representative of at least 3 separated experiments.

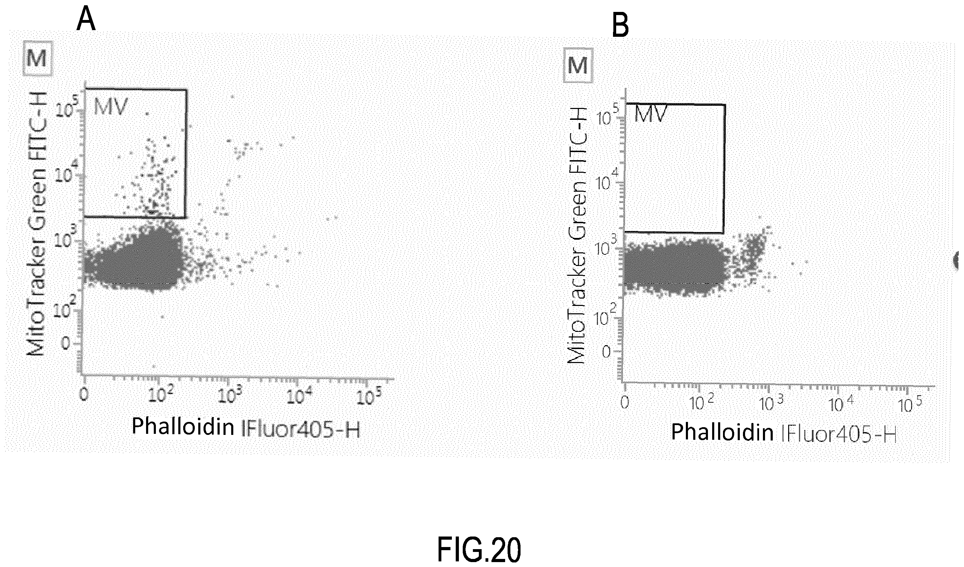

Figure 20 shows a Phalloidin-iFluor405-H/MitoTracker Green FM-FITC-H dot-plot in which microvesicles (previously obtained from the respective "MV Gate") from a sample stained by the panel described in Table 3 are represented, before (Figure 20A) or after (Figure 20B) the treatment with the Triton X-100 detergent, as described in Example 14. Data are representative of at least 3 separated experiments.

### DEFINITIONS

The term "fluorescent reagent" refers to a chemical compound or to a protein molecule, for example an antibody, conjugated to a chemical compound (fluorochrome) which upon a light excitation, is able to re-emit light at a specific wavelength.

By the term "potentiometric fluorescent reagent" according to the present invention we mean a lipophilic fluorescent and cationic reagent able to diffuse through membranes in a potential-dependent manner, accumulating in the inner membrane compartment in a proportional potential-dependent mode. Because of their lipophilic and cationic nature these reagents diffuse through plasma membranes and they are attracted by the negative internal charge of the plasma membranes. The reagents belonging to the aforementioned category are well known, commonly used by analytical techniques, such as flow cytometry or fluorescence microscopy and described in the literature, as example in Cottet-Rousselle C. et a., Cytometry A, 2011.

By the term "mitochondrial fluorescent reagent", according to the present invention, we mean a fluorescent reagent able to diffuse and accumulate inside the mitochondria in alive cells. Mitochondria fluorescent reagents are well known and are commonly used for analytical applications, for example flow cytometry or fluorescence microscopy in order to stain mitochondria in alive cells.

By the term "MitoTracker Deep Red FM" we refer to the mitochondrial potentiometric fluorescent reagent provided by Thermo Fisher Scientific with catalogue number M22426 and having the following formula: 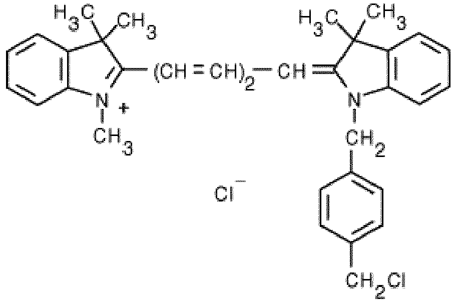 and chemical name: 1-{4-[(chloromethyl)phenyl]methyl}-3,3-dimethyl-2-[5-(1,3,3-trimethyl-1,3-dihydro-2H-indol-2-ylidene)penta-1,3-dien-1-yl]-3H-indolium chloride (CHEBI number 52131).

By the term "MitoTracker Green FM", we refer to the mitochondrial potentiometric fluorescent reagent provided by Thermo Fisher Scientific with catalogue number M7514 and having the following formula: 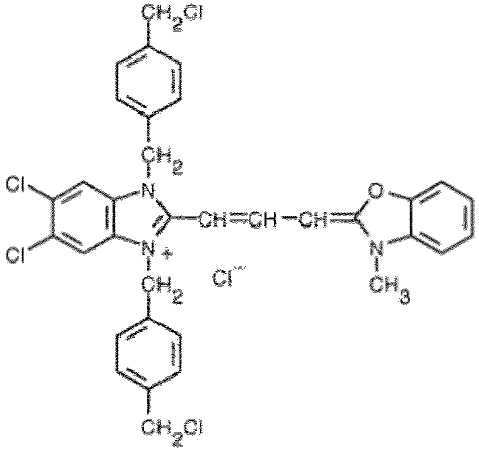 and chemical name: Benzoxazolium, 2-[3-[5,6-dichloro-1,3-bis[[4-(chloromethyl)phenyl]methyl]-1,3-dihydro-2H-benzimidazol-2-ylidene]-1-propenyl]-3-methyl-, chloride (CAS number:201860-17-5).

By the term "MitoStatus Red" we refer to the mitochondrial potentiometric fluorescent reagent provided by Becton Dickinson Biosciences (BD Biosciences) with catalogue number 64697, having a molecular weight of 504.3 g/mole, and having the following spectral characteristics: Maximum Excitation peak at 622 nm, Maximum Emission peak at 648 nm, Maximum Absorbance (UV-visible spectrum) at 660 nm.

All these three reagents are commonly used in flow cytometry techniques in order to stain mitochondria in alive cells.

By the term "whole biological fluid sample" or "whole blood sample", we mean a sample of a biological fluid or, in particular, of blood that has been drawn from a subject and kept intact, in particular it has not undergone any processing step aimed to eliminate or disrupt some of its cellular or corpuscular components, such as for example platelets, erythrocytes, leukocytes. Those treatments are for example centrifugation, ultracentrifugation, lysis of erythrocytes, freezing/thawing cycles. The aforementioned term also includes samples of a biological fluid, in particular blood, treated with anticoagulant agents, treated with agents that prevent the degradation of some of their components or diluted with solutions compatible with the used method of the analysis.

By term "control unit" refers to an electronic component which may include at least one of: a digital processor (for example including at least one element among: CPU, GPU, GPGPU), a memory system (or memories), an analogic circuit, or a combination of one or more digital processing units with one or more analogic circuits. The control unit can be "configured" or "programmed" to execute phases. For example, in the case of a control unit including one or more CPUs and one or more memories, one or more programs may be stored in appropriate memory blocks connected to the CPU (or CPUs) and/or to the GPU (or CPUs); the program or programs contain instructions which, when executed by the CPU or GPU, program or configure the control unit to perform the operations described in relation to the control unit. Alternatively, if the control unit is or includes an analogic circuitry, then the control unit circuit may be designed to include configured circuitry, in use, to process electrical signals so that it is possible to perform the steps related to the control unit. The control unit may comprise one or more digital units, for example a microprocessor type, or one or more analogic units, or a suitable combination of digital and analogic units; the control unit can be configured to coordinate all the actions needed for the execution of an instruction or instruction sets. In particular, the control unit is configured or programmed to receive at least one signal from one or more signal detectors (for example one or more optical sensors) from the flow cytometer, to process it and perform some operations based on the configuration or programming performed on the same control unit. The control unit can also be configured or programmed to control, optionally manage, the activation of one or more devices for the emission of a light beam (for example a laser) used to insist (intercept) a liquid; the control unit, by the aforesaid detectors, is then configured or programmed to receive a signal representative of a variation of the conductivity of the liquid and, as a function of this variation, perform some operations based on the configuration or programming performed on the same control unit.

By the term "subject" refers to an animal or a human, preferably human.

By the term "forward scattering parameter" (FSC, Forward-scattered light), we refer to a parameter, detected by a flow cytometer, which is the measurement of the light refracted by each single particle at narrow angles (180° ± 0-20°) (Poncelet P et al., 2015, Transfus Apher Sci. 2015 Oct; 53 (2): 110-26).

By the term "side scattering parameter" (SSC or Side-scattered light) we refer to a parameter, detected by a flow cytometer, which is the measurement of the light refracted by each single particle at wide angles (90°).

### DETAILED DESCRIPTION OF THE INVENTION

A first object of the present invention is a method for identifying, and optionally enumerating and/or classifying, microvesicles, either containing or not mitochondria, present in a biological sample, preferably whole, isolated from a subject comprising the following steps:
a) contacting said sample with at least the following fluorescent reagents:
   - a first fluorescent reagent consisting of a mitochondrial fluorescent reagent,
      preferably potentiometric, having the formula (I): 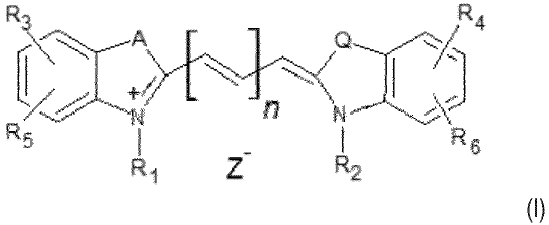
      wherein n is 0 or an integer between 1 and 3;
      R₁ and R₂ are different from each other and selected from among H and organic substituents with a molecular weight lower than 300 g/mol,
      A and Q are independently selected from among O, S, Se, C (R₇R₈), CH = CH, NR₉, wherein R₇, R₈ and R₉ are independently selected from among H and organic substituents with a molecular weight lower than 300 g/mol; R₃, R₄, R₅ and R₆ are independently selected from among H, organic substituents of molecular weight lower than 300 g/mol, or R₃ and R₄ and/or R₅ and R₆ are combined to form an additional aromatic or heteroaromatic ring, preferably a benzene ring,
      Z- is an ion selected from among halides and ClO₄⁻; and
   - a second fluorescent reagent, selected from among phalloidin and an antibody directed against CD235a conjugated to a fluorochrome group;
b) analysing the treated sample obtained in step a) and identifying the particles contained in the sample which have dimension smaller than 1 µm, preferably between 0.1 to 1 µm, positive to the detection of a fluorescence signal emitted by the aforementioned first fluorescent reagent, and negativity to the detection of a fluorescence signal emitted by the aforementioned second fluorescent reagent, thereby identifying microvesicles, either containing or not mitochondria, present in the sample.

Preferably, in the aforementioned method according to the invention, the substituents R₁ and R₂ of the first fluorescent reagent of formula (I) are selected from among H and hydrocarbon groups, optionally substituted with halogens, preferably chlorine. According to a particularly preferred realisation form, these R₁ and R₂ substituents are selected from among H and C₁-C₃ alkyl groups, among them the methyl group is particularly preferred, substituted or not substituted with halogens, preferably chlorine, or with a (chloro-C₁-C₃-alkyl) phenyl group, more preferably chloromethylphenyl. According to an even more preferred form R₁ and R₂ are selected from among C₁-C₃ alkyl groups, among them the methyl group is particularly preferred, not substituted or substituted with halogens, preferably chlorine, or with a (chloro-C₁-C₃-alkyl) phenyl group, more preferably chloromethylphenyl.

Preferably, in the aforementioned method according to the invention, the substituents R₇, R₈ and R₉ of the first fluorescent reagent of formula (I) are hydrocarbon groups, optionally substituted with heteroatoms, preferably with halogens, more preferably with chlorine. According to a particularly preferred realisation form, R₇, R₈ and R₉ are independently selected among C₁-C₃ alkyl groups, preferably methyl, not substituted or substituted with halogens, more preferably with chlorine, chloro-C₁-C₃ alkylphenyl groups.

Preferably, in the aforementioned method according to the invention, the substituents R₃, R₄, R₅ and R₆ of the first fluorescent reagent of formula (I) are hydrocarbon groups having a number of carbon atoms lower than 15, preferably less than 10, more preferably groups C₁-C₆ linear or branched alkyl, halogens, preferably chlorine, or R₃ with R₄ and/or R₅ with R₆ are combined to form an additional aromatic or heteroaromatic ring, preferably a benzene ring.

Preferably, in the aforementioned method according to the invention, Z⁻ in the first fluorescent reagent of formula (I) is Cl⁻ or I⁻.

According to a particularly preferred form of the method according to the invention, the first fluorescent reagent is selected from among MitoTracker Deep Red FM 1-{4-[(chloromethyl)phenyl]methyl}-3,3-dimethyl-2-[5-(1 ,3,3-trimethyl-1,3-dihydro-2H-indol-2-ylidene)penta-1,3-dien-1-yl]-3H-indolium chloride, MitoTracker Green FM, Benzoxazolium, 2-[3-[5,6-dichloro-1,3-bis[[4-(chloromelhyl)phenyl]methyl]-1,3-dihydro-2H-benzimidazol-2-ylidene]-1-propenyl]-3-methyl-, chloride and MitoStatus Red.

Therefore, it is particularly preferred a method to identify, and optionally enumerate and/or classify, microvesicles, containing or not containing mitochondria, present in a biological sample, preferably whole, comprising the following steps:
a) put the above mentioned sample in contact with at least:
   - a first fluorescent reagent selected from among MitoTracker Deep Red FM 1-{4-[(chloromethyl)phenyl]methyl}-3,3-dimethyl-2-[5-(1,3,3-trimethyl-1,3-dihydro-2H-indol-2-ylidene)penta-1,3-dien-1-yl]-3H-indolium chloride, MitoTracker Green FM, Benzoxazolium, 2-[3-[5,6-dichloro-1,3-bis[[4-(chloromethyl)phenyl]methyl]-1,3-dihydro-2H-benzimidazol-2-ylidene]-1-propenyl]-3-methyl-, chloride and MitoStatus Red;
   - a second fluorescent reagent, selected from among phalloidin and an antibody directed against CD235a and conjugated to a fluorochrome group;
b) analyse the treated sample obtained in step a) and identify the particles contained in the sample which have dimension smaller than 1 µm, preferably in the ranging from 0.1 to 1 µm, positive for the detection of a fluorescence signal emitted by the aforementioned first fluorescent reagent and negativity to the detection of a fluorescence signal emitted by the aforementioned second fluorescent reagent, thereby identifying microvesicles, containing or not containing mitochondria, present in the sample.

Preferably, in the method according to the invention the fluorochrome group of the second fluorescent reagent has a fluorescence emission at a wavelength different from that of the first fluorescent reagent, such as to allow the detection of the emitted signal separated from the first one. In the case the first fluorescent reagent is MitoTracker Deep Red FM, MitoTracker Green FM or MitoStatus Red selected fluorochrome groups suitable to be used for the second reagent are all fluorochrome groups detectable by means of the available optical arrangement of the instrument and that do not overlap with the fluorescence emitted by MitoTracker Deep Red FM or MitoStatus Red (which emits in the Allophycocyanin channel, APC), or that do not overlap with the MitoTracker Green FM emission fluorescence channel (which emits into the Fluorescein Isothiocyanate channel, FITC). For example, if classical optical instruments are used, and when MitoTracker Deep Red FM or MitoStatus Red are used, the fluorochrome groups suitable for the second fluorescent reagent are: FITC, PE, PerCP (or PerCP-Cy5.5), PE-Cy7, APC-Cy7 (or APC-H7), Violet 450 (or BV421), BV510 (or Violet 500). However, when MitoTracker Green FM is used, the fluorochrome groups suitable for the second fluorescent reagent are, for example, PE, PerCP (or PerCPCy5.5), APC, PE-Cy7, APC-Cy7 (or APC-H7), Violet 450 (or BV421), BV510 (or Violet 500).

The above mentioned biological fluid sample used to carry out the method according to the invention may be blood, plasma, tears, saliva, cerebrospinal fluid, synovial fluid, ascitic tumour or urine, preferably it is a blood sample. Preferably, said biological fluid sample is blood, preferably is whole.

In fact, unlike the methods used for identifying microvesicles described in the prior art, the method of the present invention can be carried out directly on a sample, taken from a patient, of a whole biological fluid, in particular whole blood, without being processed by any pre-treatment, such as centrifugation, ultracentrifugation, freezing and thawing or treated with lysing agents to remove some of the cellular or corpuscular components from the blood sample.

As it will be shown in the experimental section, microvesicles present in a blood sample remain intact for up to four hours from the collection.

Therefore, preferably, the whole blood sample used to apply the method has to be taken from a subject no longer than four hours.

Furthermore, the best storage condition is room temperature. Therefore, preferably the whole blood sample on which the method is applied has been stored at room temperature.

The biological fluid sample can be diluted before step a) with suitable solutions, for example Hepes or PBS buffer solutions or commercial solutions such as Binding Buffer 1X for Annexin V (provided by BD Biosciences). In step a) the first fluorescent reagent is added to the sample to be analysed, preferably in an amount such as to obtain a final concentration ranging from 0.05 to 6 µM.

Preferably, when the first fluorescent reagent is Mitostatus Red or the Mitotracker Deep Red FM, it is added in step a) to the sample that has to be analysed by using an amount of the reagent allowing the achievement of a final concentration in the sample equal or greather than 0.05 µM, preferably ranging from 0.05 to 5 µM, more preferably ranging from 0.80 to 1.5 µM, even more preferably ranging from 0.9 to 1.0 µM, even more preferably 0.95 µM.

Preferably, when the first fluorescent reagent is Mitotracker Green FM, it is added in step a) at a concentration greather than 3 µM, preferably ranging from 3 µM to 6 µM, more preferably ranging from 3.5 µM to 5.5 µM, even more preferably ranging from 4 to 5 µM, even more preferably 4.752 µM.

The aforementioned phalloidin conjugated to a fluorochrome group is produced by using techniques well known to the expert in the art and it is commercially available from different companies. In step a) this reagent is added to the sample of a biological fluid that has to be analysed at the concentrations and under the conditions commonly used to obtain F-actin binding.

For example, a phalloidin conjugated to a fluorochrome suitable for its use in the method of the invention is the Phalloidin-FITC provided by Sigma Aldrich with catalogue number P5282, when MitoTracker Deep Red FM, or MitoStatus Red are used as first reagent; or Phalloidin-iFluor 405 provided by Abcam with catalogue number ab176752 when MitoTracker Green FM is used as first reagent.

The aforementioned antibody directed against CD235a conjugated to a fluorochrome group is produced by techniques well known to the expert in the art and it is commercially available from different sources. In the step a) this reagent is added to the sample of a biological fluid that has to be analysed at the concentrations and under the conditions commonly used for its binding to the antigen. For example, the antibodies directed against CD235a and conjugated to a fluorochrome group suitable to be used in the method of the invention are BV421 or APC, provided by BD Biosciences with catalogue numbers 562938 or 551336, respectively.

As demonstrated in Example 10, in contrast to the traditional method for the identification of microvesicles, the method according to the invention, when carried out on samples of whole biological fluids, specifically identifies the microvesicle population present in the sample. Therefore, according to the invention, the method does not require any sample manipulation before the analysis,that, as above discussed, leads to an underestimation of the number of identified microvesicles.

Moreover, as stated in the experimental section, in contrast to traditional methods, the method according to the invention allows to identify microvesicles in the whole size range.

For example, as demonstrated in Example 10 with respect to the traditional method described in Example 10, the method according to the invention allows to identify a number of platelet derived microvesicles 200-450 times higher.

The microvesicle population identified by the method of the invention varies according to the second fluorescent reagent used in step a).

In fact, when the phalloidin is used as second fluorescent reagent, the method according to the invention identifies microvesicles from any cell origin that are present in the biological fluid sample.

When the antibody directed against CD235a is used as second fluorescent reagent, being the marker used to exclude positive events from the count, the method according to the invention identifies a microvesicle population that does not contain the subpopulation of erythrocyte-derived vesicles, which usually constitutes about 2% of total microvesicles.

Therefore, the method according to the invention, using as second reagent an antibody direct against CD235a identifies preferably between 97% and 99%, preferably 98% of the microvesicles identified by the same method that uses as second fluorescent reagent the phalloidin.

According to a preferred way of realization, in the method according to the invention the step b) is performed by a flow cytometer, in which the emission of a fluorescent signal is stimulated and detected from the above mentioned first and second fluorescent reagents and the parameters of diffusion of the light of the particles present in the sample are detected. Preferably, the flow cytometer is a multiparametric flow cytometer (FACS), characterized by a configuration allowing the detection of at least two fluorescence parameters.

According to an alternative preferred realization, in the method based on the invention, the step b) is performed in an imaging flow cytometer (ImageStream, Amnis), in which it is stimulated and detected the emission of a fluorescent signal from the above mentioned first and second reagents and the light diffusion parameters of the particles present in the sample are detected. According to a further alternative preferred realization, in the method according to the invention, the step b) is performed by an instrument which records the Brownian motion of the microvesicles to determine their size, and it is associated with an ultramicroscope which allows to visualize the microvesicles in suspension (Nanoparticle Tracking Analysis, NanoSight Ltd).

In the aforementioned preferred ways of realization, before to carry out the step b), the sample treated in step a) is preferably diluted in order to avoid swarm phenomena, in which different particles are detected as a single particle. Preferably, the sample is diluted in a ratio ranging from 1:1 to 1:200 ratio, more preferably the sample is diluted 1:140, with an appropriated solution, preferably the same used to dilute the biological fluid sample in step a).

When step b) is performed in a flow cytometer, sample particles with microvesicle sizes are identified by the correspondence between the forward scattering and side scattering parameters of particles having sizes smaller than 1 µm, preferably in the range 0.1-1 µm.

Preferably, before the analysis of the biological fluid sample, forward scattering and side scattering parameters are set on the flow cytometer by using standard samples, which are fluorescent beads having dimension in the microvesicular dimensional range (0.1 µm-1 µm). For example, products suitable to be used for this application are the commercial product Megamix Plus FSC and the commercial product Megamix Plus SSC, provided by Biocytex.

Preferably, the previously mentioned step b), carried out by a flow cytometer, includes the following steps:
b1) establish a region (gate) of analysis based on the correspondence between the parameters of forward scattering and side scattering of particles having sizes smaller than 1 µm, more preferably ranging from 0.1 to 1 µm;
b2) acquire fluorescence data and forward scattering and side scattering parameters by the flow cytometer for all the events of the analysed sample;
b3) identify among the events with light refraction parameters in the aforementioned region (gate) established in step b1), particles resulting positive for the detection of the fluorescent signal emitted by the first reagent and negative for the detection of the fluorescent signal emitted by the second reagent, in this way identifying microvesicles containing or not containing mitochondria, present in the sample.

Preferably, in the aforementioned step b), according to the invention the total microvesicles present in the analysed biological fluid sample is also enumerated by counting the number of events in the sample that satisfy the above mentioned parameters of light scatter and fluorescence positivity and negativity and the absolute numbers of microvesicles per unit of biological fluid volume are calculated. Alternatively, the absolute numbers of total microvesicles can be obtained through any other validated flow cytometry method. For example, a procedure suitable to be used in flow cytometry to determine the number of microvesicles *per* volume unit of the analysed biological fluid, involves the use of fluorescent beads, at a known number or concentration that has to be mixed to the sample that has to be analysed (Venditti A. et al., 1999, Bone Marrow Transplant, 24 (9): 1019-27).

The method of the invention also allows to classify the microvesicles as different subpopulations, based on their cell origin. As it will be discussed below, the composition of the microvesicle population provides useful information for diagnostic and prognostic purposes.

Microvesicles are characterized by the expression on the plasma membrane surface of specific antigens of the cell population from which they stem. Therefore, the method of the invention can include a further step c), preferably between step b) and step d), in which the identified microvesicles are sub-classified as different subpopulations on the basis of their cell origin through the detection of the cell membrane markers indicative of specific cell types. Preferably, such a step c) is performed by a flow cytometer.

According to the method of the invention, preferably in step a) the biological fluid sample, optionally diluted, is placed in contact with the above mentioned first and second fluorescent reagents, as well as with a panel of one or more further fluorescent reagents able to bind cell membrane markers indicative of specific cell types, preferably of endothelial cells, platelets, leukocytes or activated cells, and in step c) the emission of a fluorescent signal is detected from each of the aforementioned fluorescent reagents and microvesicles of each subpopulation are identified and enumerated on the basis of their positivity and/or negativity to the detection of fluorescent signals emitted by the different fluorescent reagents of the panel.

Preferably, steps b) and c) are carried out by a flow cytometer.

The positivity and/or negativity of the different detected fluorescence signals emitted by the fluorescent reagents of the panel, indicates the presence or absence of cellular membrane markers, indicative of specific cell types and therefore allow the identification of the origin of each detected microvesicle.

Moreover, in this case, preferably the different fluorescent reagents of the panel exhibit fluorescence emissions at different wavelengths also compared to the first and the second reagents and therefore a separate detection of the signals emitted by each fluorescent reagent of the panel is possible.

Preferably, the panel of further fluorescent reagents includes Annexin V conjugated to a fluorochrome group and antibodies conjugated to fluorochrome groups and directed against CD41a, CD31 or CD45 antigens, and in step c) platelet-derived microvesicles are identified by their simultaneous positivity to the detection of CD41a and CD31; leukocyte-derived microvesicles are identified for their positivity to CD45 detection, endothelial-derived microvesicles are identified for the positivity to the detection of CD31 and their negativity to the detection of CD41a and CD45 signals; microvesicles that expose phosphatidylserine (reported as a sign of damage or activation of the cells from which they stem) are identified for the positivity to the detection of a fluorescent signal emitted by Annexin V.

The above fluorescent reagents are known and commonly used by the expert in the fields. Furthermore, they are commercially available and are used in the step a) at the concentrations and incubation conditions suitable for obtaining a binding to the cellular marker against which they are directed.

For example, an antibody directed against CD41a, suitable for its use in the method according to the invention is the one provided by BD Biosciences with catalogue number 555467, an antibody directed against CD31 suitable for its use in the method according to the invention is the one provided by BD Biosciences with catalogue number 563651, an antibody directed against CD45 suitable for its use in the method according to the invention is the one provided by BD Biosciences with catalogue number 563204, an Annexin V suitable for its use in the method according to the invention is the one provided by BD Biosciences with catalogue number 561431.

During the following flow cytometry analysis, in order to avoid artefact deriving from the *background* produced by the aggregation of the antibody molecules or reagent of the staining panel, preferably the stock solutions of the different fluorescent reagents used in the step a) of the method of the present invention have to be centrifuged, preferably at 21000 g for 12 minutes, before they are added to the biological fluid sample.

The method according to the present invention can also include a further step d), preferably after step b) and, when present, step c), in which identified microvesicles are isolated and collected. When a flow cytometer is used in the analysis of a biological sample, the isolation and collection of microvesicles takes place by a cell sorter module.

In this type of instrument, the hydrodynamic focus of the analysed particles is realized by the use of an isotonic buffer which flow through a very small hole, producing, in this way, a very thin stream of drops. Vesicles or cells are injected into this flow one at a time.

The laser light source is focused on the flow, and sensitive light detectors collect the optical signals emitted by the cells or microvesicles. As soon as the light and the fluorescence signals generated by the cells are collected, the program of the instrument defines whether the cell or the microvesicle has to be separated or not from the rest of the sample population, according to the criteria established by the user. Cells or microvesicles can be separated in a sterile environment.

At this step, the total population of microvesicles present in the biological fluid sample, preferably blood, or a specific microvesicle subpopulation identified through the presence of cell membrane marker patterns, as described above, can be isolated and collected.

Preferably, when the method of the present invention is carried out by a flow cytometer, step b), and, when present, c) and/or d) are carried out and controlled by the flow cytometer control unit.

The number and the cellular origin of the microvesicles present in a sample of a biological fluid can provide useful diagnostic and prognostic indications, as some pathologies have been associated with alteration of the number or composition of the different microvesicle sub-populations.

Therefore, a second object of the present invention is a method for the prognosis and/or diagnosis of a pathology in a subject, that encloses the identification, enumeration and/or classification of microvesicles, either containing or not mitochondria, in a biological fluid sample from the subject, according to the first object of the invention and the subsequent correlation between the number of microvesicles present in the sample and/or the presence and/or quantity of one or more microvesicle subpopulations in the sample with a specific diagnosis or prognosis. A third object of the present invention is a kit for identifying, and optionally enumerating and/or classifying and/or isolating microvesicles, containing or not containing mitochondria, present in a biological sample, preferably whole, isolated from a subject, by means of a method according to the first object of the invention as above described, comprising at least:
- a first fluorescent reagent consisting of a mitochondrial fluorescent reagent,
   preferably potentiometric, as above described, having the following formula (I): 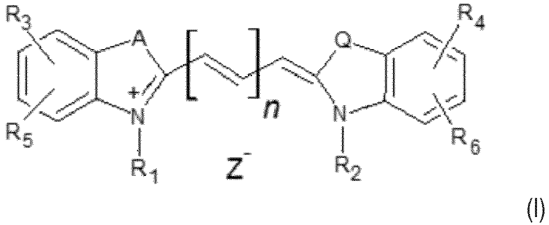
   wherein n is 0 or an integer between 1 and 3;
   R₁ and R₂ are different from each other and selected from among H and organic substituents with a molecular weight lower than 300 g/mol,
   A and Q are independently selected from among O, S, Se, C (R₇R₈), CH = CH, NR₉, in which R₇, R₈ and R₉ are independently selected from among H and organic substituents with a molecular weight lower than 300 g/mol;
   R₃, R₄, R₅ and R₆ are independently selected from among H, organic substituents of molecular weight lower than 300 g/mol or R₃ and R₄ and/or R₅ and R₆ are combined to form an additional aromatic or heteroaromatic ring, preferably a benzene ring;
   Z- is an ion selected from halides and ClO₄⁻; and
- a second fluorescent reagent as above defined selected from a phalloidin or an antibody directed against CD235a conjugated to a fluorochrome group.

Preferably, in the above mentioned kit according to the invention, the substituents R₁ and R₂ of the first fluorescent reagent of the formula (I) are selected from among H and hydrocarbon groups optionally substituted with halogens, preferably chlorine. According to a particularly preferred realization form, the aforesaid substituents R₁ and R₂ are selected from H and C₁-C₃ alkyl groups, among them the methyl is particularly preferred, not substituted or substituted with halogens, preferably chlorine, or with a group (chloro-C₁-C₃-alkyl)phenyl, more preferably chloromethylphenyl. According to an even more preferred form R₁ and R₂ are selected from among C₁-C₃ alkyl groups, among them methyl is particularly preferred, not substituted or substituted with halogens, preferably chlorine, or with a group (chloro-C₁-C₃-alkyl)phenyl, more preferably chloromethylphenyl.

Preferably, in the previously mentioned kit according to the invention, the substituents R₇, R₈ and R₉ of the first fluorescent reagent of formula (I) are hydrocarbon groups, optionally substituted with heteroatoms, preferably with halogens, more preferably with chlorine. According to a particularly preferred realization R₇, R₈ and R₉ are independently selected among C₁-C₃ alkyl groups, preferably methyl, not substituted or substituted with halogens, more preferably chlorine, or with groups chloro-C₁-C₃ alkylphenyl.

Preferably, in the method kit according to the invention, the substituents R₃, R₄, R₅ and R₆ of the first fluorescent reagent of the formula (I) are hydrocarbon groups having a number of carbon atoms lower than 15, preferably less than 10, more preferably groups C₁-C₆ are linear or branched with alkyl, halogens, preferably chlorine, or R₃ with R₄ and/or R₅ with R₆ are combined to form an additional aromatic or heteroaromatic ring, preferably a benzene ring.

Preferably, in the above mentioned kit according to the invention, Z⁻ in the first fluorescent reagent of formula (I) is Cl⁻ or I⁻.

According to a particularly preferred form, the kit according to the invention comprises a first fluorescent reagent selected from among MitoTracker Deep Red FM (1-{4-[(chloromethyl)phenyl]methyl}-3,3-dimethyl-2-[5-(1, 3,3-trimethyl-1,3-dihydro-2H-indol-2-ylidene) penta-1,3-dien-1-yl] -3H-indolium chloride), MitoTracker Green FM, Benzoxazolium, (2- [3- [5,6-dichloro-1,3-bis [[4- (chloromethyl) phenyl] methyl] -1,3-dihydro-2H-benzimidazol-2-ylidene]-1-propenyl]-3-methyl- , chloride), and MitoStatus Red.

Preferably, the previously mentioned kit also comprises a panel of one or more fluorescent reagents able to bind cell membrane markers identifying specific cellular types, preferably endothelial cells, platelets, leukocytes or apoptotic cells. Preferably, this panel comprises Annexin V conjugated to a fluorochrome group and the antibodies directed against CD41a, CD31 or CD45 antigens and conjugated to fluorochrome groups. Preferably, the fluorescent groups of the fluorescent reagents of the kit are selected in a way that allows that signal emissions of the different fluorescent reagents occur at different wavelengths.

The fluorescent reagents present in the kit are preferably in a lyophilized form.

The fluorescent reagents present in the kit are preferably mixed together in a same package, preferably a test container, in which the sample that has to be analysed is then added.

Moreover, optionally, the kit includes additional components to carry out the method according to the invention, for example a further container containing fluorescent beads at a known number or concentration for the enumeration of microvesicles and/or an instruction manual.

The present examples are provided for illustrative and not limitative purpose of the invention.

### EXPERIMENTAL EXAMPLES

### Microvesicles identification in whole blood samples

### Example 1- Optimization of flow cytometry scatter parameters

In the present experiment FACSVerse flow cytometers (BD Biosciences) has been used, equipped with three lasers and eight fluorescence parameters, and data have been analysed using FACSuite v 1.0.5, FACSDiva v 6.1.3 (BD Biosciences) and FlowJo v 8.8.6 (TreeStar, Ashland, OR, USA) software. Similar results to those here described have been obtained by FACSCanto II or FACSAria III cytometer (BD Biosciences).

Before the analysis of the samples, dedicated beads of known size that cover the whole dimensional range described for microvesicles (0.1-1 µm) have been used to optimize side scattering (SSC) and forward scattering (FSC) parameters of the flow cytometry, and in particular, related height signals (SSC-H, FSC-H) which identify particles having microvesicle dimensions.

In particular, fluorescent beads have been used that emit in the fluorescein isothiocyanate (FITC) channel: MegamixPlus SSC (dimensions: 0.16, 0.20, 0.24 and 0.50 µm) and Megamix Plus FSC (dimensions: 0.10 µm, 0.30 µm, 0.50 µm and 0.90 µm) (Biocytex, Marseille, France).

Parameters regulation was carried out on the basis of the FSC signal collection angle, as reported in the literature (Edwin Van Der Pol et al., Pharmacol Rev, 64 (676-705), 2012).

In particular, the setting of scatter parameters was carried out using both Megamix-Plus SSC, and Megamix-Plus FSC beads. In this way, scatter parameters can be optimized in order to study microvesicles (MV) within a region in which the size remains constant with respect to those defined by the setting obtained by acquiring the beads. In particular, in order to identify the region containing the microvesicles of smaller size (in particular the events smaller than the wavelength of the incident light, i.e. events smaller than 488 nm), Megamix-Plus SSC beads were used, while in order to identify the upper limit of the region containing the microvesicles (events larger than the wavelength of the incident light, i.e. 488 nm), Megamix-Plus FSC beads were used.

The following steps were carried out:
The commercial suspension of Megamix-Plus SSC beads has been mixed for at least 10 seconds and 500 µl of the suspension was added to a flow cytometry tube.

On the flow cytometer, have been selected:
- the height signal (H) for SSC, FSC and for all fluorescence parameters;
- logarithmic and/or bi-exponential visualization for FSC-H, SSC-H and for all fluorescence parameters.

For the acquisition of the beads, it was used:
- a FITC-H/SSC-H dot-plot, with the bi-exponential display for both parameters;
- a FSC-H/SSC-H dot-plot, with bi-exponential display for both parameters;
   The threshold has been set on the FITC channel and the FITC photomultiplier was adjusted in order to detect the FITC fluorescence of all the beads and the acquisition of the beads has started obtaining the pattern shown in Figure 1A. In Figure 1A, the regions related to each of the different bead populations (0.16, 0.20, 0.40, 0.50 micrometres) were individually selected on the dot plot FITC-H/SSC-H . The voltage of the SSC-H has been modified so that SSC MegaMix Plus beads appeared on a FSC-H/SSC-H dot-plot as reported in Figure 1C.

The MegaMix Plus SSC beads (20,000 events) were then acquired.

Then we proceeded with the acquisition of MegaMix Plus FSC beads in order to identify the limit for the vesicles of the same size or bigger than the incident light length (from 488 nm to 1 µm) using the same procedure above described for a bead suspension of MegaMix Plus FSC.

The acquisition of Megamix Plus FSC beads gave the pattern shown in Figure 1B. Also, in this case, the regions related to each of the different populations of beads on the FITC-H/SSC-H dot-plot were selected individually in Figure 1B. The FSC parameter has also been modified so that MegaMix Plus SSC beads appear on a FSC-H/SSC-H dot plot, as shown in Figure 1D.

The Megamix-Plus FSC beads were acquired (20,000 events).

On the obtained FSC-H/SSC-H dot-plot (Figures 1C and 1D), a region containing all the MegaMixPlus SSC and all the MegaMixPlus FSC beads has been drawn, and it was defined as "MV Gate" (Figures 1C and D) that identifies the region in which we expect to find the events characterized by the sizes reported for microvesicles. To avoid the exclusion of microvesicles with lowest scatter signals, no lower threshold and gating limits were imposed with respect to scatter parameters. All dot plots and histograms obtained from the analyses carried out on blood samples described below are identified in the "MV" region.

### Example 2- Verification of instrumental sensitivity on scatter parameters

The possibility to identify, on the flow cytometers FACSVerse, FACS Canto II and FACSAria III, the biological events with microvesicle size, ranging from 0.1 to 1 µm, in the "MV gate" has been verified by the acquisition of fluorescent liposomes having diameters ranging from 0.05 µm to 1 µm, obtained as described in the paper from Cristiano MC et al., Colloids and Surfaces B: Biointerfaces, 2017; 150: 408-416. The advantage in the use of liposomes (whose membranes have a composition similar to that of biological membrane) relies on the fact that they have a refractive index similar to that of microvesicles.

Liposomes have been labelled with Rhodamine, MitoTracker Deep Red FM, MitoStatus Red (1 µl 0.2 mM, 45 minutes, at 4 ° C, in the dark), or MitoTracker Green FM (1 µl of 1 mM, 45 minutes, 4 ° C, in the dark) and unlabelled and labelled liposomes have been acquired. Results obtained are shown in Figure 2, that represents the signal detected for the Rhodamine-loaded liposomes (Figure 2A), MitoTracker Deep Red FM (Figure 2B) or MitoStatus Red (Figure 2C), or MitoTracker Green FM (Figure 2D), compared to the same unstained liposomes. On the basis of the obtained results, it is clear that the here described probes (MitoTracker Deep Red FM, MitoStatus Red and MitoTracker Green FM) because of their lipophilic properties are able to bind the lipids that compose the liposomal envelopes.

Moreover, liposomes have been acquired using a FACSVerse flow cytometer and data have been analysed using the FACSuite v 1.0.5 software (BD Biosciences).

The different probes used to stain liposomes allowed us to place a threshold on each related fluorescence channel (PE for Rhodamine, APC for MitoTracker Deep Red FM and for MitoStatus Red, FITC for MitoTracker Green FM), and to analyse the dimensional placement of the produced liposomes on the respective FSC-H/SSC-H dot-plot.

As can be observed in Figure 3, regardless from the fluorochrome used to stain liposomes, and therefore regardless from the channel on which the acquisition threshold has been set, in the "MV Gate" region, identified by MegaMix Plus SSC and FSC beads, liposomes of sizes ranging from 0.05 µm to1 µm can be distinguished. Therefore, data confirm that the optimization of the physical parameter regulation used for the identification of events having microvesicles size is correct.

### Example 3- Sample preparation and staining

Peripheral blood samples were obtained from 203 healthy volunteers after they have signed the informed consent (approved by the local ethics committee) and in agreement with the guidelines established by the II Helsinki Declaration and relevant current legislation, by using 21G needles. Blood samples were obtained by using two tubes containing 3.8 % sodium citrate as an anticoagulant. The first drawn tube (the first 3 ml of blood) was discarded to minimize the effects of vascular damage induced by the venepuncture. As it is shown in Example 13, microvesicles in the blood samples remain stable if the analysis is carried out within 4 hours from blood collection.

Reagents from the different panels, containing MitoTracker Deep Red FM (panel of Table 1), MitoStatus Red (panel of Table 2) or MitoTracker Green FM (panel of Table 3), as below described have been added to 195 µl of 1X Binding Buffer (BD Biosciences); 5 µl of blood were then added and finally incubated for 45 minutes at room temperature, in the dark.

During the subsequent phase of flow cytometry analysis, in order to avoid the production of artefacts, deriving from the *background* produced by the aggregation of the antibody or reagent molecules of the labelling panel, when liquid stock solutions were used (not lyophilized) of the different reagents, they have been centrifuged at 21,000 g for 12 minutes before they were added to blood samples. Each antibody or dye was titrated under the assay conditions; different dilutions were tested and the ones that allowed to obtain the highest signal for the positive population and the lowest signal for the negative population (best signal to noise ratio) were selected. 500 µl of 1X binding buffer (BD Biosciences) were then added to the labelled sample.

The further final dilution of blood sample before to carry out flow cytometry analysis was established in order to avoid the *swarm effect,* by which distinct particles very close each other could be simultaneously illuminated by the cytometer laser and therefore could be considered as single big particle.

In whole blood samples (N=203), acquired with the same bead and liposome voltages, a population of events with scatter values lower than those detected for platelets was highlighted. In the area identified by the MV Gate ("MV Gate", established on the basis of the size of beads and liposomes), it is observed the population of events characterized by the microvesicle expected scatter parameters for the MV and, the acquisition of whole blood samples by using the same voltages of liposomes and beads, highlights that this gate excludes, indeed, platelets (gate "PLT") which are characterized by higher scatter parameters than those established for microvesicles (see for example Figure 4, representative of all samples analysed by this method).

### Example 4- Identification and characterization of microvesicles in the samples by a fluorescent reagent panel containing MitoTracker Deep Red FM.

### Blood samples were obtained from 20 healthy volunteers (males and females) aged in the range 19-50, as above described.

Samples were stained and prepared for the analysis as described in Example 3, using the reagent panel listed in Table 1; the amount of the used reagents is indicated here below (Table 1).

| **Table 1** | | | | | |
|---|---|---|---|---|---|
| ***Marker*** | ***Fluorochrome*/*Reagent*** | ***Vendor*** | ***Clone*** | ***Catalogue number*** | ***Amount of reagent*/*antibody per test*** |
| Mito Tracker Deep Red FM | (Equivalent of APC) | Thermo Fisher Scientific | - | M22426 | **1 µl di 0.2 mM** |
| Phalloidin-FITC | (Equivalent of FITC) | Sigma-Aldrich | - | P5282 | **0.2 µl di 0.5 mg/ml** |
| CD41a | R-phycoerythrin (PE) | BD Biosciences | HIP8 | 555467 | **2.5 µl** |
| Annexin V | PerCP-Cv5.5 | BD Biosciences | - | 561431 | **0.25 µl** |
| CD31 | PE-Cy7 | BD Biosciences | WM59 | 563651 | **0.5 µl** |
| CD45 | BV510 | BD Biosciences | HI30 | 563204 | **1 µl** |
| CD235a | BV421 | BD Biosciences | GA-R2 (HIR2) | 562938 | **5 µl** |

Samples were then analysed by flow cytometry, acquiring 1 x 10⁶ events/sample (instrument: FACSVerse, BD Biosciences with three laser configuration and eight fluorescence parameters).

In particular, light diffusion and fluorescence data were acquired at the specific different emission wavelengths of each single reagent; the threshold was placed on the allophicocyanin channel (APC, acquisition channel for MitoTracker Deep Red FM). In order to avoid the exclusion from the acquisition of the microvesicles with the lowest scatter signals, no limit was established with respect to the scatter parameters.

For the evaluation of the non-specific fluorescence of all reagents, with the exception of the MitroTracker Deep Red FM, Fluoresce Minus One (FMO) controls were used. For the evaluation of the non-specific fluorescence on emission channel of MitoTracker Deep Red FM, a sample prepared as above described was used, to which a detergent solution has been added (Triton X-100, 1% final concentration, for an hour before the acquisition). Compensation was calculated using CompBeads (BD Biosciences) and single-stained fluorescent samples. Data were analysed using FACSDiva v 6.1.3, FACSuite v 1.0.5 (BD Biosciences) and FlowJo v 8.8.6 (treeStar, Ashland, OR, USA) software.

In detail, as shown in Figure 5, which is representative of the analysis carried out on all the analysed samples, once the "MV gate" region has been drawn, adjusted as described in Example 1, within this region were identified the events, that have been analysed using the gating strategy shown in Figure 5.

In detail, the total microvesicle population has been identified by its positivity to MitoTracker Deep Red FM fluorescence staining and its negativity to Phalloidin staining (H signals) (Figure 5A).

Identified microvesicles were then analysed for belonging to different subpopulations, according to their cellular origin through the analysis of the reactivity to the different used markers. In particular, platelet-derived microvesicles were identified on the basis of their positivity to CD41a and CD31 (Figure 5B, PLT_MV), endothelial-derived microvesicles based on their positivity to CD31 and negativity to the staining for CD41a and CD45 (Figure 5C, Endo_MV); leukocyte-derived microvesicles were identified on the basis of their positivity to CD45 (Figure 5C); microvesicles derived from activated or damaged cells were identified by their binding to Annexin V (Figure 5D).

Absolute numbers of total microvesicles and belonging of each analysed population have been obtained through a volumetric counting method. In particular, by means of a device, which is part of the instrument equipment, the FACSVerse allows the absolute counting of events (and therefore of microvesicles) with respect to the volume of the analysed sample.

In figure 6 are represented the control samples Triton X-100 and FMO carried out for the fluorescence assessment.

Results obtained show that MitoTracker Deep Red FM, in combination with phalloidin and with the scatter parameters, allows to define a population of microvesicles circulating in the non-manipulated peripheral blood, and to distinguish them with respect to debris and other cellular figurative elements of blood.

The use, in association with the two above mentioned compounds, of an appropriate panel of reagents able to recognize specific surface antigenic pattern, allows, to identify and count microvesicles from platelet, leukocyte or endothelial origin.

### Example 5- Identification and characterization of microvesicles in the samples by a fluorescent reagent panel containing Mitostatus Red

Blood samples have been obtained from 120 healthy volunteers (males and females) aged in the range 19-70, as above described.

Samples have been stained as described in Example 3, using the fluorescent reagent panel listed in Table 2; the amount of the used reagents is indicated here below (Table 2).

| **Table 2** | | | | | |
|---|---|---|---|---|---|
| ***Marker*** | ***Fluorochrome*/*Reagent*** | ***Vendor*** | ***Clone*** | ***Catalogue number*** | ***Amount of reagent*/*antibody per test*** |
| MitoStatus Red | (Equivalent of APC) | BD Biosciences | - | 564697 | **1 µl di 0.2 mM** |
| Phalloidin-FITC | (Equivalent of FITC) | Sigma-Aldrich | - | P5282 | **0.2 µl di 0.5 mg/ml** |
| CD41a | PE | BD Biosciences | HIP8 | 555467 | **2.5 µl** |
| Annexin V | PerCP-Cy5.5 | BD Biosciences | - | 561431 | **0.25 µl** |
| CD31 | PE-Cy7 | BD Biosciences | WM59 | 563651 | **0.5 µl** |
| CD45 | BV510 | BD Biosciences | HI30 | 563204 | **1 µl** |
| CD235a | BV421 | BD Biosciences | GA-R2 (HIR2) | 562938 | **5 µl** |

Once stained, samples have been then analysed by flow cytometry, acquiring 1 x10⁶ events/sample (instrument: FACSVerse, BD Biosciences with three laser configuration and eight fluorescence parameters). The threshold was placed on the allophicocyanin channel (APC, acquisition channel of MitoStatus Red). In order avoid the exclusion from the acquisition of the microvesicles with the lowest scatter signals, no limit was established with respect to the scatter parameters.

For the evaluation of the non-specific fluorescence, of all fluorescence reagents, with the exception of the MitoStatus Red, Fluoresce Minus One (FMO) controls were used. For the evaluation of the non-specific fluorescence on emission channel of MitoStatus Red, a sample prepared as above described was used, to which a detergent solution has been added (Triton X-100, 1% final concentration, for an hour before acquisition). Compensation was calculated using CompBeads (BD Biosciences) and single-stained fluorescent samples. Data have been analysed using FACSDiva v 6.1.3 (BD Biosciences), FACSuite v 1.0.5 (BD Biosciences) and FlowJo v 8.8.6 (treeStar, Ashland, OR, USA) software.

Fluorescence data identified within the MV gate region have been analysed following the gating strategy of Figure 7, representative of the analysis performed on all the samples. In detail, the total microvesicle population has been identified by its positivity to MitoStatus Red fluorescence staining and its negativity to phalloidin staining (H signals) (Figure 7A).

Identified microvesicles have been then analysed for belonging to different subpopulations, according to their cellular origin through the analysis of the reactivity to the different used markers, as already described in Example 4.

In particular, platelet-derived microvesicles have been identified on the basis of their positivity to CD41a and CD31 staining (Figure 7B, PLT_MV), endothelial-derived microvesicles were identified on the basis of their positivity to CD31 and negativity to CD41a and CD45 staining (Figure 7C, Endo_MV); leukocyte-derived microvesicles were identified on the basis of their positivity to CD45 (Figure 7C); microvesicles derived from activated or damaged cells have been identified by their binding to Annexin V (Figure 7D, AnnexV).

In figure 8 are represented the control samples Triton X-100 and FMO carried out for the fluorescence assessment.

Absolute numbers of total microvesicles and belonging to each analysed population have been obtained through a volumetric counting method. In particular, by means of a device, which is part of the instrument equipment, FACSVerse allows the absolute counting of events (and therefore of microvesicles) with respect to the volume of the analysed sample. Even in this case, the absolute numbers of total microvesicles belonging to each analysed population were obtained by a volumetric count method as above described.

### Example 6- Identification and classification of microvesicles by a panel of fluorescent reagents containing Mitotracker Green FM

Blood samples were obtained from 72 subjects (males and females) aged in the range 39-80.

Samples have been obtained and stained as described in Example 3, using the fluorescent reagent panel listed in Table 3, the amount of the used reagents is indicated here below (Table 3).

| **Table 3** | | | | | |
|---|---|---|---|---|---|
| ***Marker*** | ***Fluorochro me*/*Reagent*** | ***Vendor*** | ***Clone*** | ***Catalogue number*** | ***Amount of reagent*/*antibody per test*** |
| MitoTracker Green FM | (Equivalent of FITC) | Thermo Fisher Scientific | - | M7514 | **1 µl di 1mM** |
| Phalloidin iFluor 405 | IFluor 405 | AbCam | | ab176752 | **0.2 µl del test** |
| CD41a | PE | BD Biosciences | HIP8 | 555467 | **2.5 µl** |
| Annexin V | PerCP-Cy5.5 | BD Biosciences | - | 561431 | **0.25 µl** |
| CD31 | PE-Cy7 | BD Biosciences | WM59 | 563651 | **0.5 µl** |
| CD45 | BV510 | BD Biosciences | HI30 | 563204 | **1 µl** |
| CD235a | APC | BD Biosciences | GA-R2 (HIR2) | 551336 | **5 µl** |

For the acquisition by flow cytometry (FACSVerse, BD Biosciences), the threshold was placed on the Fluorescein Isothiocyanate channel (FITC, acquisition channel of Mitotracker Green FM). In order avoid the exclusion from the acquisition the MV with the lowest scatter signals, no limit was established with respect to the scatter parameters.

The gates for all fluorescent reagents, with the exception of the Mitotracker Green FM, have been established on the basis of the "Fluoresce Minus One" controls (FMO). For the evaluation of the non-specific fluorescence on the emission channel of Mitotracker Green FM, a sample prepared as above described was used, to which a detergent solution (Triton X-100 1% for one hour before the acquisition) was added. Compensation was calculated using CompBeads (BD Biosciences) and single-stained fluorescent samples.

Data were analysed using FACSDiva v 6.1.3 (BD Biosciences), FACSuite v 1.0.5 (BD Biosciences) and FlowJo v 8.8.6 (treeStar, Ashland, OR, USA) software.

Fluorescence data identified within the MV gate region have been analysed by the gating strategy reported in Figure 9. In detail, the total microvesicle population was identified by its positivity to the Mitotracker Green FM staining and its negativity to phalloidin staining.

The identified microvesicles have been then analysed for their different subpopulation, according to their cellular origin through the analysis of the reactivity to the different used markers, as already described in Examples 4 and 5.

In figure 10 are represented the Triton X-100 and FMO control samples acquired for the assessment of the fluorescence.

Even in this case, absolute numbers of total microvesicles and each analysed sub-population have beenobtained by a volumetric counting method, as above described.

### Example 7- Characterization of the phalloidin positive population

The analysis of the population of events identified in Examples 4, 5 and 6 into the "MV Gate" region and which are positive for phalloidin, revealed that the majority of this population is positive for CD235a expression, as shown in Figure 11A, which is representative of data obtained from the sample tested in the three examples above mentioned. These events (Phallodin+/CD235a+) have been then separated by instrumental cell sorting (Nozzle 70 µm, obtained purity: 95-98%), and observed by transmission electron microscopy: results show that these are erythrocytic "ghosts" (Figure 11B, even in this case it is representative of data obtained from the tested sample in the aforementioned three examples). Therefore, microvesicles can be distinguished from this population by the negativity to the binding to CD235a (instead of the negativity to the phalloidin binding), when there is no interest in the analysis of the erythrocyte-derived microvesicles.

### Example 8 - Comparative analysis of the obtained results

Four samples which were tested in the Examples 4, 5 and 6 were obtained from the same patients. Figure 12 shows the average number of total microvesicles identified in those blood samples from patients tested in parallel in the Examples 4, 5 and 6. As can be observed, the different three panels of fluorescent dyes used lead to obtain microvesicle counts which overlap each other.

Dot-plots obtained for these samples in Examples 4 and 5, compared to those obtained in Example 6 allow to highlight that a better resolution of microvesicles in whole blood can be obtained.

### Example 9- Validation of the dimensions of the identified microvesicles.

Microvesicles, identified by applying the procedures described in Examples 4, 5 and 6 on peripheral blood samples from three healthy individuals (3 samples for each subject) using a FACSAria III with a 70 µm nozzle (BD Biosciences), were separated by instrumental sorting, achieving a purity > 97%.

Purified microvesicles were then analysed by Dynamic Light Scattering (DLS), transmission electron microscopy or ImageStream (AMNIS Seattle, USA), to confirm their size.

DLS analysis has revealed that the microvesicles identified by the procedure according to the invention have size ranging from 0.1 to 0.9 µm. Data also demonstrate that the size of events identified by the described protocol allows to identify microvesicles and to distinguish them with respect to other figurative elements, in peripheral blood, and these events have sizes that exactly overlap to those reported in the literature for microvesicles. By the ImageStream analysis (60X magnification) both microvesicles and beads of known size MegaMix-Plus SSC and FSC beads have been acquired. Laser power parameters have not been modified among the different microvesicle and bead acquisition. Beads have been used as a reference for the size. Figure 13 shows the image obtained for 3 different microvesicles, with progressively increasing dimensions from the top to the bottom and highlights that the elements separated by instrumental cell sorting have dimensions ranging from 0.1 to 0.5 µm. The analysis of the microvesicles identified and isolated with the method according to the invention has also highlighted that, as can be observed in Figure 14, pure elements have vesicular features.

### Example 10 - Comparison with traditional methods

Numbers of platelet-derived microvesicles, obtained by application of the classical protocol based on a pre-analytical separation step (R.Lacorix et al., Journal of Thrombosis and Haemostasis, 11: 1190-1193, 2012), and numbers of platelet-derived microvesicles obtained by analysing the whole blood (of the same individuals) by the application of the protocol here described have been compared.

Peripheral blood samples from 3 enrolled healthy volunteers, have been stained and analysed using the protocol herein described in Examples 4, 5 or 6 or as described in the paper by Lacroix et al., 2012.The latter involves two consecutive centrifugation steps of the peripheral blood at 2500 x g for 15', without brake, at room temperature to separate microvesicles from other blood figurative elements and the staining of the supernatant. In details, the supernatant (platelet free plasma-PFP) was recovered and the contained vesicles have beenstained by the panels described in Table 1, Table 2, or Table 3.

Whole blood samples were acquired as described in Examples 4, 5 and 6; PFP samples have been acquired as described by Lacroix et al. (2012).

Numbers of platelet-derived microvesicles (PLT_MV) identified by the positivity to CD41a have been obtained by means of volumetric count as described in Examples 4, 5 or 6, or, in the case of PFP samples, as described by Lacroix et al., 2012.

As shown in Figure 15, by the standard protocol application (light grey bar) can be observed a consistent and statistically significant loss of events that are PLT-derived microvesicles (CD41a+), with respect to the same determination carried out on whole peripheral blood using the protocol here described and applied to the same individuals (left bar, dark grey).

### Example 11- Action mechanism of MitoTracker Deep Red FM, MitoStatus Red and MitoTracker Green FM

In order to verify the percentage of containing mitochondria microvesicles, three samples of peripheral blood, obtained from three different subjects, have been collected and stained following the procedure already described in Example 3 by MitoTracker Deep Red FM (at the concentration reported in Table 1) or MitoStatus Red (at the concentration reported in Table 2), in combination with JC1 (4 µM) and CD235a (5 µl).

Samples were then acquired as described in Example 4 or in Example 5. Microvesicles have been identified at first by drawing a gate on the region that, on the dot-plot SSC-H/FSC-H, identifies microvesicles ("MV Gate"), then all the events CD235a negatives, which are considered microvesicles have been identified. The following populations have been, then, identified, according to their classical scatter parameters: red blood cells (RBC); platelets (PLT); leukocytes (Leuko), used as control populations.

In details, the percentage of events that emit fluorescence in the red channel (sign of the accumulation of JC1 in the mitochondria) was verified in relation to the four identified populations.

As shown in Figure 16, the percentages of events containing mitochondria (red and green JC1) obtained with the panel containing the MitoTracker Deep Red FM and with the MitoStatus Red, are overlapping and are the following ones:
- 13-61% of total microvesicles identified by the method of the invention using MitoTracker Deep Red FM or MitoStatus Red
- 0.0-12% of the RBC population above described
- 92-99% of the PLT population above described
- 96-100% of the LEUKO population above described

As shown in Figure 16, approximately the 13-61% of the total population of the identified microvesicles resulted also positive to JC1-Red. Consistently with the literature, red blood cells do not do not display fluorescence in the red channel since they do not contain mitochondria, while the majority of platelets (> 90%) and leukocytes (> 95%) displays also positivity to JC1 red (platelets and leucocytes contain mitochondria).

These results demonstrate that only a subset of microvesicles identified by the protocol here described contains mitochondria. Therefore, MitoTracker Deep Red FM and MitoStatus Red and MitoTracker Green FM are able to identify the circulating population of microvesicles, independently from their known ability to recognize mitochondria-containing cells.

On the basis of the characteristics of those molecules, it has been hypothesized that they are able to identify the circulating population of microvesicles, through a dual mechanism: the binding of the plasma membrane lipids (as demonstrated in example 2) and a potential-dependent diffusion. In order to verify this last hypothesis, 3 samples of peripheral blood have been treated for 15 minutes with a high concentration (50 µM) of carbonyl cyanide-m-chlorophenylhydrazone (CCCP, Sigma Aldrich Corporation, St Louis, MO, USA), a ionophore that allows to break down the Δψ(Sigma Aldrich).

After these treatments samples were stained by Mitotracker Deep Red FM and phalloidin (at the concentrations reported in Table 1, and by the protocol described in Example 4) or by MitoStatus Red and phalloidin (at the concentrations reported in Table 2, and by the protocol described in Example 5), or by MitoTracker Green FM and phalloidin (at the concentrations shown in Table 3, and using the protocol described in Example 6), and then analysed by flow cytometry.

The mean fluorescence intensity value of MitoTracker Deep Red FM, or MitoStatus Red or MitoTracker Green FM was detected in the MV compartment before and after the CCCP treatment (at the above indicated concentration). We have therefore calculated the percentage of reduction of MitoTracker Deep Red FM, MitoStatus and MitoTracker Green FM fluorescence, in the compartment identified by the region "MV" and we have obtained that:
- The CCCP (50 µM) induces a 13-14% reduction of the fluorescence of the MitoTracker Deep Red FM in the compartment identified by the respective "MV" region (see EXAMPLE 4);
- The CCCP (50 µM) induces a 42-66%reduction of the fluorescence of MitoStatus Red in the compartment identified by the respective "MV" region (see EXAMPLE 5);
- The CCCP (50 µM) induces a 31-34% reduction of the fluorescence of MitoTracker Green FM in the compartment identified by the respective "MV" region (see EXAMPLE 6).

These data indicate that MitoTracker Deep Red FM, MitoStatus Red and MitoTracker Green FM, bind to membrane phospholipids, as well as accumulate inside the microvesicles as these microvesicles present a trans-membrane potential; for this reason microvesicle appear fluorescent and distinguishable from the debris. This mechanism allows the identification of the microvesicle compartment in the whole blood, although only a subset of microvesicles contains mitochondria.

### Example 13- Optimal storage conditions for the analysis of microvesicles in whole blood samples

In order to verify how and for how long it is possible to store whole blood samples for identification and for the count of microvesicles, 3 samples of whole blood were drawn from 3 different subjects and stored at 37° C, at RT (20-22° C) or at 4° C. Whole blood samples were then processed as described in Examples 4, 5 and 6 (using the panel described in Table 1, the panel in Table 2 or the panel in Table 3), at the following time points: at the collection time ("Baseline"), after 2 hours (h), after 4h, after 6h and after 24h from the collection. It was therefore calculated the average percentage of deviation from the "baseline" of the total microvesicle counts obtained at the different storage temperatures and results, shown in Figure 17 (17A: storage at 37° C; 17B: storage at RT; 17C: storage at 4° C) demonstrate that the sample remains stable, both in terms of numbers and morphology of the microvesicle compartment if it is analysed within 4h from collection. The best storage condition is the room temperature. At this temperature, sample stability up to 24 hours is observed.

### Example 14- Control of the accuracy of the signal associated to MitoTracker Deep Red FM, MitoStatus Red or MitoTracker Green FM.

In order to demonstrate that the signals identified in the region named "MV" are real and not associated with various artefacts, 3 samples of whole blood have been drawn from 3 different individuals and processed as described in Examples 4, 5 or 6, acquiring before and after their treatement with a detergent (Triton X-100 1% final concentration, for one hour). As shown in Figures 18-20, the population defined into the "MV" region by MitoTracker Deep Red FM, by MitoStatus Red or by MitoTracker Green FM (Figures 18A, 19A and 20A, respectively) disappears after the treatment with the detergent (Figure 18B, 19B and 20B, respectively).

## Claims

1. Method for identifying the microvesicles, either containing or not mitochondria, present in a biological fluid sample, preferably whole, isolated from a subject comprising the following steps:
a) contacting said sample with at least the following fluorescent reagents:
- a first fluorescent reagent, consisting of a mitochondrial fluorescent reagent, preferably potentiometric, having the formula (I): 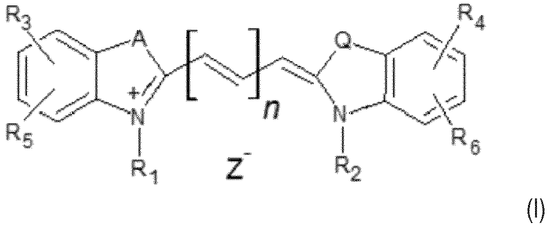
wherein n is 0 or an integer between 1 and 3;
R₁ and R₂ are different from each other and selected from among H and organic substituents with a molecular weight lower than 300 g/mol,
A and Q are independently selected from among O, S, Se, C (R₇R₈), CH = CH, NR₉, wherein R₇, R₈ and R₉ are independently selected from among H and organic substituents with a molecular weight lower than 300 g/mol;
R₃, R₄, R₅ and R₆ are independently selected from among H, organic substituents of molecular weight lower than 300 g/mol or R₃ and R₄ and/or R₅ and R₆ are combined to form an additional aromatic or heteroaromatic ring, preferably a benzene ring;
Z- is an ion selected from among halides or ClO₄⁻; and
- a second fluorescent reagent selected from among phalloidin and an antibody directed against CD235a conjugated to a fluorochrome group;
b) analysing the treated sample obtained in step a) and identifying the particles contained in the sample which have dimension smaller than 1 µm, preferably between 0.1 to 1 µm, positive to the detection of a fluorescence signal emitted by the aforementioned first fluorescent reagent, and negative for the fluorescence signal emitted by the aforementioned second fluorescent reagent, thereby identifying the microvesicles, either containing or not mitochondria, present in the sample.

2. Method according to claim 1, wherein in said first fluorescent reagent of formula (I), the substituents R₁ and R₂ are selected from among H and hydrocarbon groups optionally substituted with halogens, preferably chlorine, preferably the aforesaid substituents R₁ and R₂ are selected from among H and C₁-C₃ alkyl groups, preferably methyl, not substituted or substituted with halogens, preferably chlorine, or with a group (chloro-C₁-C₃-alkyl)phenyl, preferably chloromethylphenyl, more preferably the aforesaid substituents R₁ and R₂ are selected among C₁-C₃ alkyl groups, preferably methyl, not substituted or substituted with halogens, preferably chlorine, or with a group (chloro-C₁-C₃-alkyl)phenyl, more preferably chloromethylphenyl and/or the aforesaid substituents R₇, R₈ and R₉ are hydrocarbon groups, optionally substituted with heteroatoms, preferably with halogens, more preferably with chlorine, more preferably R₇, R₈ and R₉ are independently selected among C₁-C₃ alkyl groups, preferably methyl, not substituted or substituted with halogens, more preferably chlorine, or with a chloro-C₁-C₃ alkylphenyl group and/or the aforesaid substituents R₃, R₄, R₅ and R₆ are hydrocarbon groups with a number of carbon atoms less than 15, preferably less than 10, more preferably they are linear or branched C₁-C₆ alkyl groups, halogens, preferably chlorine, or R₃ and R₄ and/or R₅ and R₆ taken together form an additional aromatic or heteroaromatic ring, preferably a benzene ring and/or Z⁻ is preferably Cl⁻ or I⁻.

3. Method according to claim 1 or 2, wherein the first fluorescent reagent is selected from MitoTracker Deep Red FM (1-{4-[(chloromethyl)phenyl]methyl}-3,3-dimethyl-2-[5-(1,3,3-trimethyl-1,3-dihydro-2H-indol-2-ylidene)penta-1,3-dien-1-yl]-3H-indolium chloride), MitoTracker Green FM, Benzoxazolium, (2-[3-[5,6-dichloro-1,3-bis[[4-(chloromethyl) phenyl]methyl]-1,3-dihydro-2H-benzimidazol-2-ylidene]-1-propenyl] -3-methyl- , chloride), and MitoStatus Red.

4. Method according to claims 1 to 3, wherein the aforementioned biological fluid sample is a whole biological fluid sample and/or it is selected from blood, plasma, tears, saliva, cerebrospinal fluid, synovial fluid, ascitic tumour or urine, preferably it is a whole blood sample.

5. Method according to claims 1 or 4, wherein step b) is carried out in a flow cytometer, in which the emission of a fluorescent signal is stimulated and detected from the aforementioned first and second fluorescent reagents and the diffusion parameters of the light of the particles contained in the sample are detected.

6. Method according to Claim 5, wherein the particles present in the sample having the dimensions microvesicles are identified by the correspondence with the forward scattering and side scattering parameters of particles having dimensions smaller than 1 µm, preferably comprised between 0.1 to 1 µm.

7. Method according to claims 5 or 6, wherein said step b) comprises:
b1) establishing an analysis region (gate) based on the correspondence with the forward scattering and side scattering parameters of particles smaller than 1 µm, more preferably between 0.1-1 µm;
b2) acquiring fluorescence data and forward scattering and side scattering parameters from the cytometer for all the events of the analysed sample;
b3) identifying, within the events with light refraction parameters, in the aforementioned region (gate) set in step b1), the particles present in the sample positive to the detection of the fluorescent signal emitted by the first fluorescent reagent and negative to the detection of the fluorescent signal emitted by the second fluorescent reagent, thereby identifying the microvesicles, either containing or not mitochondria, present in the sample.

8. Method according to claims from 5 to 7, wherein in the step b) the total microvesicles are also enumerated by counting the number of events in the sample that satisfy the above parameters of light scattering and of positivity and negativity to fluorescence and/or wherein, after step b), in a further step d) the identified microvesicles are isolated and collected by the flow cytometer provided with a sorting module.

9. Method according to claims from 1 to 8, comprising a further step c), between step b) and step d), in which the identified microvesicles are classified as different subpopulations on the basis of their cell origin through the detection of the expression of specific cellular membrane markers indicative of specific cell types.

10. Method according to claim 9, wherein in step a) the biological fluid sample is also contacted with a panel of further fluorescent reagents, comprising Annexin V conjugated to a fluorophore group and antibodies conjugated to fluorochrome groups and directed against the antigens CD41a, CD31 or CD45, and in step c) platelet-derived microvesicles are identified for positivity to CD41a and CD31 detection; leukocyte-derived microvesicles are identified for positivity to CD45 detection; endothelial-derived microvesicles are identified for positivity to the detection of CD31 and negativity to the detection of a signal CD41a and CD45; microvesicles stemming from activated or damaged cells are identified for positivity to the detection of a fluorescent signal emitted from Annexin V.

11. Method for the prognosis and/or the diagnosis of a pathology in a subject, comprising identifying, enumerating and/or classifying the microvesicles, either containing or not mitochondria, in a biological fluid sample, preferably whole, isolated from the subject through the method according to claims from 1 to 10 and the subsequent correlation of the number of microvesicles present in the sample and/or of the presence and/or quantity of one or more microvesicle subpopulations present in the sample with a diagnosis or prognosis.

12. Kit for the identifying, and optionally enumerating and/or classifying, and/or isolating microvesicles, either containing or not mitochondria, present in a biological fluid sample, preferably whole, preferably whole blood, isolated from a subject comprising
- a first fluorescent reagent, consisting of a mitochondrial fluorescent reagent, preferably potentiometric, having the formula (I): 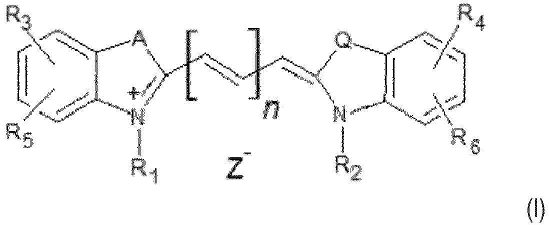
wherein n is 0 or an integer between 1 and 3;
R₁ and R₂ are different from each other and selected from among H and organic substituents with a molecular weight lower than 300 g/mol;
A and Q are independently selected from among O, S, Se, C (R₇R₈), CH = CH, NR₉, wherein R₇, R₈ and R₉ are independently selected from among H and organic substituents with a molecular weight lower than 300 g/mol;
R₃, R₄, R₅ and R₆ are independently selected from among H, organic substituents of molecular weight lower than 300 g/mol or R₃ and R₄ and/or R₅ and R₆ are combined to form an additional aromatic or heteroaromatic ring, preferably a benzene ring;
- Z⁻ is an ion selected from among halide or ClO₄⁻; and
- a second fluorescent reagent selected from among a phalloidin and an antibody directed against CD235a conjugated to a fluorochrome group.

13. Kit according to claim 12, comprising a container comprising a mixture of the above fluorescent reagents, preferably in a lyophilized form, and, optionally, a further container containing a known number/concentration of fluorescent beads for the enumeration of microvesicles.
